# EUROPEAN PATENT SPECIFICATION

(11) **EP 1 831 172 B1**
(45) Date of publication and mention of the grant of the patent: **18.02.2009**
(21) Application number: 04816679.7
(22) Date of filing: 28.12.2004
(51) Int. Cl.: C07D 215/34, A61K 31/47, A61P 25/28

(54) **SUBSTITUTED CARBAMIC ACID QUINOLIN-6-YL ESTERS USEFUL AS ACETYLCHOLINESTERASE INHIBITORS**
SUBSTITUIERTE CARBAMINSÄURECHINOLIN-6-YLESTER, DIE SICH ALS ACETYLCHINOLYLESTERASEINHIBITOREN EIGNEN
ESTERS D'ACIDE CARBAMIQUE QUINOLINE-6-YLE SUBSTITUES UTILES EN TANT QU'INHIBITEURS DE L'ACETYLCHOLINESTERASE

(43) Date of publication of application: 12.09.2007
(73) Proprietor: Council of Scientific and Industrial Research, New Delhi 110 001 (IN)
(72) Inventor: SHAKYA, Neeraj Cental Drug Research Institute, Uttar Pradesh (IN); FATIMA, Zeeshan Cental Drug Research Institute, Uttar Pradesh (IN); NATH, Chandishwar Cental Drug Research Institute, Uttar Pradesh (IN); SAXENA, Anil, Kumar Cental Drug Research Institute, Uttar Pradesh (IN)
(74) Representative: Fairbairn, Angus Chisholm
(86) International application number: PCT/IN2004/000427
(87) International publication number: WO 2006/070394

(56) References cited:
- EP-A- 1 382 598
- US-A- 3 997 542
- US-A- 4 472 404

## Description

### Field of the invention

The present invention relates to novel derivatives of quinolinyl carbamic acid esters. The present invention particularly relates to new substituted carbamic acid quinolinyl esters useful as acetylcholinesterase inhibitors, which show potent antiacetylcholinesterase activity and have potential therapeutic use for prevention or cure of Alzheimer's disease, senile dementia or memory disturbance. The present invention particularly relates to compounds of the formulae 1 and 2 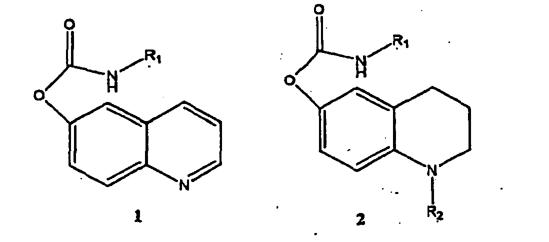 wherein R₁ = alkyl, aryl, 2-chlorophenyl, 3-bromo-phenyl, 4-bromophenyl, 4-chloro-3-trifluoromethyl-phenyl; R₂=H, alkyl, aralkyl.

### Background of the invention

Alzheimer's disease (AD) is a chronic neurodegenerative disorder. The characteristic symptom of AD in the patient is gradual decline in cognitive function. Despite several approaches to the treatment of this disorder, still there is no well-approved therapy to check the progression of AD except the inhibition of acetylcholinesterase (AChE). The currently available drugs used for AD include donepezil (Bryson, H.M.; Benfield, P. Drugs Aging 1997, 10, 234), galanthamine (Fulton, B.; Benfield, P, Drugs Aging, 1996, 9, 60), tacrine (Summers, W.K.; Majovski, L.V.; Marsh, G.M.; Tachiki, K.; Kling, A.; The New England Journal of Medicine, 1946, 315, 1241; Wagstaff, A.J.; McTavish, D. Drugs Aging 1994, 4, 510), rivastigmine (Spencer, C.M.; Noble, S. Drugs Aging 1998,13, 391) and memantine (Möbius. H. J.; Stöffler, A.; Graham, S.M. Drugs of Today, 2004, 40, 685). The major side effects associated with most of these drugs are liver toxicity, headache, fatigue, dizziness, nausea, vomiting, loss of appetite, joint pain, insomnia etc. Apart from these drugs several other molecules have shown potent activity in different test models including animal models. Some of these are xanthostigmine (Rampa,A.; Piazzi,L; Bulleti, F.; Gobbi, S.; Bisi, A; Bartolini, M.; Andrisano, V.; Cavrini, V.; Cavalli, A.; Recanatini, M.; Valenti, P. J. Med. Chem., 2001, 44, 3810), physostigmine (Moeller H.J.; Hampel H.; Hegerl U.; Schmitt W. and Walter K. Pharmacopsychiatry 1999, 32, 99), phenserine (Al-Jafari A. A., Kamal, M. A., Greig, N. H. J Phsiol., 1998, 92, 402), Huperzine-A (Kozikowsky, A. P.; Campiani, G.; Sun, L. Q.; Wang, S.; Saxena, A; Doctor, B. P. J. Am. Chem. Soc., 1996, 118, 11357), bis-tacrine (Pang, Y. P.; Quiram, P; Jelacio, T.; Hong, F.; Brimjoin, S. J. Biol Chem., 1996,271, 23646) etc. As the average age is increasing all over the world, and so the AD (18 million people worldwide; 66% people in developing countries), there is an urgent need to identify novel candidate molecule for drug development.

### Objects of the invention

The main object of the present invention is to provide novel molecules incorporating quinoline flanked on one side by carbamic acid ester and on the other side hydrogen or alkyl like methyl or aralkyl like benzyl groups that exhibit better therapeutic efficacy to treat dementia of Alzheimer's type.

It is a further object of the invention to provide compounds useful for the treatment or prevention of senile dementia of Alzheimer's type, vascular dementia, alcoholic demntia, dementia associated with neurological disorders such as epilepsy, neoplasm and post-trauma and dementia related with behavioral disorders like depression.

It is another object of the invention to provide compounds useful for the treatment or prevention of atony of the smooth muscle of the intestinal tract (paralytic ileus) and atony of urinary bladder.

It is another object of the invention to provide compounds useful for the treatment or prevention of glaucoma and myasthenia gravis.

### Summary of the invention

Accordingly the present invention provides a quinoline derivative represented by formula 1 and 2 below: 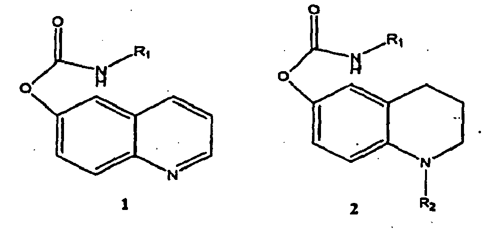 wherein R₁ = alkyl, aryl, 2-chlorophenyl, 3-bromo-phenyl, 4-bromophenyl, 4-chloro-3-trifluoromethyl-phenyl; R₂=H, alkyl, aralkyl.

In one embodiment of the invention, the substituted carbamic acid quinolinyl esters obtained are selected from the group consisting of:
**1a.** hexyl-carbamic acid quinolin-6-yl ester
**1b.** heptyl-carbamic acid quinolin-6-yl ester
**1c.** (2-chloro-phenyl)-carbamic acid quinolin-6-yl ester
**1d.** (3-bromo-phenyl)-carbamic acid quinolin-6-yl ester
**2a.** hexyl-carbamic acid 1, 2, 3, 4-tetrahydro-quinolin-6-yl ester
**2b.** heptyl-carbamic acid 1, 2, 3, 4-tetrahydro-quinolin-6-yl ester
**2c.** (3-bromo-phenyl)-carbamic acid 1,2,3,4-tetrahydro-quinolin-6-yl ester
**2d.** (2-chloro-phenyl)-carbamic acid 1, 2, 3, 4-tetrahydro-quinolin-6-yl ester
**2e.** (4-chloro-3-trifluoromethyl-phenyl)-carbamic acid 1, 2, 3, 4-tetrahydro-quinolin-6-yl ester
**2f.** (4-bromo-phenyl)-carbamic acid 1, 2, 3, 4-tetrahydro-quinolin-6-yl ester
**2g.** heptyl-carbamic acid 1-benzyl-1, 2, 3, 4-tetrahydro-quinolin-6-yl ester
**2h.** hexyl-carbamic acid 1-benzyl-1, 2, 3, 4-tetrahydro-quinolin-6-yl ester
**2i.** (2-chloro-phenyl)-carbamic acid 1-benzyl-1, 2, 3, 4-tetrahydro-quinolin-6-yl ester
**2j.** (3-bromo-phenyl)-carbamic acid 1-benzyl-1, 2, 3, 4-tetrahydro-quinolin-6-yl ester
**2k.** (4-chloro-3-trifluoromethyl-phenyl)-carbamic acid 1-benzyl-1, 2, 3, 4-tetrahydro-quinolin-6-yl ester
**2l.** (4-bromo-phenyl)-carbamic acid 1-benzyl- 1, 2, 3, 4-tetrahydro-quinolin-6-yl ester
**2m.** hexyl-carbamic acid 1-methyl-1, 2, 3, 4-tetrahydro-quinolin-6-yl ester
**2n.** heptyl-carbamic acid 1-methyl-1, 2, 3, 4-tetrahydro-quinolin-6-yl ester
**2o.** (2-chloro-phenyl)-carbamic acid 1-methyl-1, 2, 3, 4-tetrahydro-quinolin-6-yl ester
**2p.** (3-bromo-phenyl)-carbamic acid 1-methyl-1, 2, 3, 4-tetrahydro-quinolin-6-yl ester
**2q.** (4-chloro-3-trifluoromethyl-phenyl)-carbamic acid 1-methyl-1, 2, 3, 4-tetrahydro-quinolin-6-yl ester
**2r.** (4-bromo-phenyl)-carbamic acid 1-methyl-1, 2, 3, 4-tetrahydro- quinolin-6-yl ester

The present invention also provides a process for the synthesis of a quinoline derivative represented by formula 1 and 2 below: 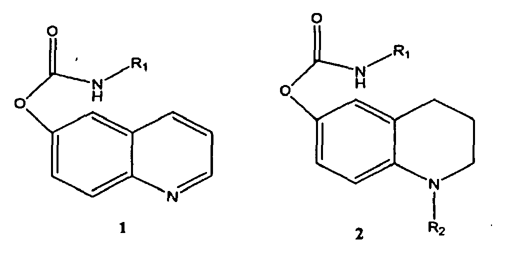 wherein R₁ = alkyl, aryl, 2-chlorophenyl, 3-bromo-phenyl, 4-bromophenyl, 4-chloro-3-trifluoromethyl-phenyl; R₂=H, alkyl, aralkyl, the process comprising reacting a substituted phenol with an isocyanate in the presence of a base and at least one organic solvent using a base and at least one organic solvent to obtain the corresponding carbamic acid ester (carbamates) of formulae 1 or 2.

In one embodiment of the invention, R₁ is selected from the group consisting of hexyl and heptyl.

In another embodiment of the invention, R₁ is selected from the group consisting of 2- chloro, 3- bromo, 4-bromo and 4-chloro-3-trifluoromethyl-phenyl.

In another embodiment of the invention, R₂ is selected from the group consisting of methyl and benzyl.

In another embodiment of the invention, the base used is selected from an organic or an inorganic base.

In another embodiment of the invention, the solvent is selected from the group consisting of ether, tetrahydrofuran (THF), dimethylformamide (DMF), dioxane, dichloromethane and chloroform.

In another embodiment of the invention, the base is selected from the group consisting of sodium hydride, sodium hydroxide, triethylamine and pyridine.

In yet another embodiment of the invention, the reaction is carried out at a temperature in the range of -10°C to 80°C and for a period between half an hour to 100 hours.

In another embodiment of the invention, the reaction is carried out in the presence of a catalyst selected from the group consisting of sodium iodide and potassium iodide.

In another embodiment of the invention the molar ratio of substituted phenol to isocyanate is in the range of 1:1 to 1:1.2.

In another preferred embodiment of the invention, the organic solvent is present in an amount in the range of 1.0 ml to 10 ml per mmol of the reactants.

In another embodiment of the invention, the compounds of formulae
**2g.** heptyl-carbamic acid 1-benzyl-1, 2, 3, 4-tetrahydro-quinolin-6-yl ester
**2h.** hexyl-carbamic acid 1-benzyl-1; 2, 3, 4-tetrahydro-quinolin-6-yl ester
**2i.** (2-chloro-phenyl)-carbamic acid 1-benzyl-1, 2, 3, 4-tetrahydro-quinolin-6-yl ester
**2j.** (3-bromo-phenyl)-carbamic acid 1-benzyl-1, 2, 3, 4-tetrahydro-quinolin-6-yl ester
**2k** (4-chloro-3-trifluoromethyl-phenyl)-carbamic acid 1-benzyl-1, 2, 3, 4-tetrahydro-quinolin-6-yl ester
**21.** (4-bromo-phenyl)-carbamic acid 1-benxyl-1, 2, 3, 4-tetrahydro-quinolin-6-yl ester
**2m.** hexyl-carbamic acid 1-methyl-1, 2, 3, 4-tetrahydro-quinolin-6-yl ester
**2n.** heptyl-carbamic acid 1-methyl-1, 2, 3, 4-tetrahydro-quinolin-6-yl ester
**2o.** (2-chloro-phenyl)-carbamic acid 1-methyl-1, 2, 3, 4-tetrahydro-quinolin-6-yl ester
**2p.** (3-bromo-phenyl)-carbamic acid 1-methyl-1, 2, 3, 4-tetrahydro-quinolin-6-yl ester
**2q.** (4-chloro-3-trifluoromethyl-phenyl)-carbamic acid 1-methyl-1, 2, 3, 4-tetrahydro-quinolin-6-yl ester
**2r.** (4-bromo-phenyl)-carbamic acid 1-methyl-1, 2, 3, 4-tetrahydro- quinolin-6-yl ester are obtained from **2a-f**
**2a.** hexyl-carbamic acid 1, 2, 3, 4-tetrahydro-quinolin-6-yl ester
**2b.** heptyl-carbamic acid 1, 2, 3, 4-tetrahydro-quinolin-6-yl ester
**2c.** (3-bromo-phenyl)-carbamic acid 1,2,3,4-tetrahydro-quinolin-6-yl ester
**2d.** (2-chloro-phenyl)-carbamic acid 1, 2, 3, 4-tetrahydro-quinolin-6-yl ester
**2e.** (4-chloro-3-trifluoromethyl-phenyl)-carbamic acid 1, 2, 3, 4-tetrahydro-quinolin-6-yl ester
**2f.** (4-bromo-phenyl)-carbamic acid 1, 2, 3, 4-tetrahydro-quinolin-6-yl ester
by first reacting compounds **2a-1** with an alkyl or aralkyl halide of formula RX wherein R is at least methyl or benzyl group and X is selected from chloro, bromo and iodio using a solvent selected from the group consisting of dimethylformamide, tetrahydrofuran and dioxane, in the presence of an organic or inorganic base selected from the group consisting of sodium hydride, sodium hydroxide, triethylamine and pyridine and at a temperature ranging between -10°C to 37°C for a period of 1 hour to 12 hours in the presence or absence of a catalyst sodium iodide or potassium iodide.

In another embodiment of the invention, compounds of formulae **2a-f** are synthesized from **2m-r** using Pd-C 5-10% catalyst in a solvent selected from group consisting of ethanol and methanol in an amount of 15-25 ml per mmol of compound, by applying hydrogen pressure in the range of 50-60 psi for a period between 4-12 hours at room temperature.

In another embodiment of the invention, the phenol is reacted with an isocyanate to obtain corresponding carbamate which is then reduced with Ni- Al alloy/KOH/ethanol to give corresponding 1, 2, 3, 4 - tetrahydro derivatives of formula **2a-1.**

In another embodiment of the invention, the phenol is reacted with an isocyanate to obtain corresponding N-benzyl derivatives **2m-r** which are then debenzylated using 5% or 10% Pd-CH₂ in ethanol or methanol as solvent to obtain the corresponding debenzylated carbamates of formulae **2a-f,** which are then N-methylated using MeI to give compounds of formulae **2m-r.**

In another embodiment of the invention, compounds of formulae 2a-I are methylated in the presence of a solvent selected from the group consisting of tetrahydrofuran, dioxane and dimethylformamide and at a temperature ranging from 10 to 37°C, for between 3 hours to 48 hours.

The present invention also comprises the use for the treatment of hypofunctioning of cholinergic system in a subject suffering from hypo functioning of cholinergic system, of a pharmaceutically effective amount of compound of formulae 1 or 2 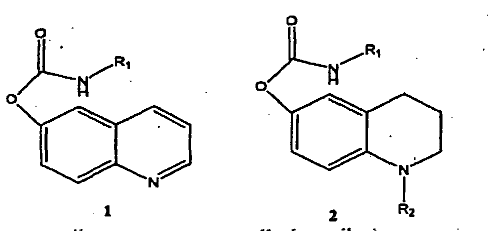 wherein R₁ = alkyl, aryl, 2-chlorophenyl, 3-bromo-phenyl, 4-bromophenyl, 4-chloro-3-trifluoromethyl-phenyl; R₂=H, alkyl, aralkyl.

In one embodiment of the invention, the hypofunctioning of cholinergic system occurs in the peripheral or central nervous system of the subject.

In another embodiment of the invention, the hypofunctioning of cholinergic system results in atony of smooth muscle of intestinal tract, atony of urinary bladder, glaucoma, myasthenia gravis and cognitive behaviour dysfunction of the subject.

In another embodiment of the invention, the subject is a mammal.

In yet another embodiment of the invention, the mammal is a human being.

### Description of figures:

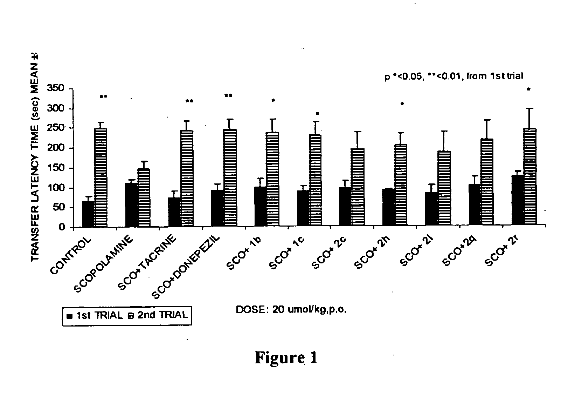

Figure 1 represents scopolamine induced deficit (dementia/amnesia) in mice Passive Avoidance Test.

### Detailed description of the invention

The main objects of the present invention are:
1. provide novel molecules incorporating quinoline flanked on one side by carbamic acid ester and on the other side hydrogen or alkyl like methyl or aralkyl like benzyl groups that exhibit better therapeutic efficacy to treat dementia of Alzheimer's type.
2. provide a method treating Alzheimer's disease. In formulae 1 and 2, the R₁ is alkyl group like hexyl, heptyl, etc.; is aryl group, or is substituted phenyl like trifluromethyl and halophenyl wherein halo is chloro, bromo etc. The R₂ group is like methyl, benzyl etc.
3. provide useful compounds for the treatment or prevention of senile dementia of Alzheimer's type.
**4.** provide useful compounds for the treatment or prevention of vascular dementia.
**5.** provide useful compounds for the treatment or prevention of alchoholic dementia.
**6.** provide useful compounds for the treatment or prevention of dementia associated with neurological disorders such as epilepsy, neoplasm and post-trauma.
**7.** provide useful compounds for the treatment or prevention of dementia related with behavioral disorders like depression.
**8.** provide useful compounds for the treatment or prevention of atony of the smooth muscle of the intestinal tract (paralytic ileus).
**9.** provide useful compounds for the treatment or prevention of atony of urinary bladder.
**10.** provide useful compounds for the treatment or prevention of glaucoma.
**11.** provide useful compounds for the treatment or prevention of myasthenia gravis.

The above objects of the invention are achieved by novel pharmacologically active substances specifically substituted carbamic acid quinolinyl esters of the formula 1 and 2, 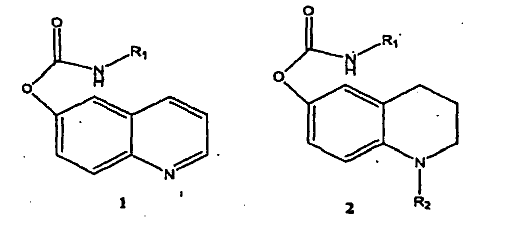 where R₁ = alkyl, aryl, 2-chlorophenyl, 3-bromo-phenyl, 4-bromophenyl, 4-chloro-3-trifluoromethyl-phenyl; R₂=H, alkyl, aralkyl, Representative compounds include:
**1a.** hexyl-carbamic acid quinolin-6-yl ester
**1b.** heptyl-carbamic acid quinolin-6-yl ester
**1c.** (2-chloro-phenyl)-carbamic acid quinolin-6-yl ester
**1d.** (3-bromo-phenyl)-carbamic acid quinolin-6-yl ester
**2a.** hexyl-carbamic acid 1, 2, 3, 4-tetrahydro-quinolin-6-yl ester
**2b.** heptyl-carbamic acid 1, 2, 3, 4-tetrahydro-quinolin-6-yl ester
**2c.** (3-bromo-phenyl)-carbamic acid 1,2,3,4-tetrahydro-quinolin-6-yl ester
**2d.** (2-chloro-phenyl)-carbamic acid 1,2,3,4-tetrahydro-quinolin-6-yl ester
**2e.** (4-chloro-3-trifluoromethyl-phenyl)-carbamic acid 1, 2, 3, 4-tetrahydro-quinolin-6-yl ester
**2f.** (4-bromo-phenyl)-carbamic acid 1, 2, 3, 4-tetrahydro-quinolin-6-yl ester
**2g.** heptyl-carbamic acid 1-benzyl-1, 2, 3, 4-tetrahydro-quinolin-6-yl ester
**2h.** hexyl-carbamic acid 1-benzyl-1, 2, 3,4-tetrahydro-quinolin-6-yl ester
2i. (2-chloro-phenyl)-carbamic acid 1-benzyl-1,2,3,4-tetrahydro-quinolin-6-yl ester
**2j.** (3-bromo-phenyl)-carbamic acid 1-benzyl-1, 2, 3, 4-tetrahydro-quinolin-6-yl ester
**2k**.(4-chloro-3-trifluoromethyl-phenyl)-carbamic acid 1-benzyl-1, 2, 3, 4-tetrahydro-quinolin-6-yl ester
**21.** (4-bromo-phenyl)-carbamic acid 1-benzyl-1, 2, 3, 4-tetrahydro-quinolin-6-yl ester
**2m.** hexyl-carbamic acid 1-methyl-1, 2, 3, 4-tetrahydro-quinolin-6-yl ester
**2n.** heptyl-carbamic acid 1-methyl-1, 2, 3, 4-tetrahydro-quinolin-6-yl ester
**2o.** (2-chloro-phenyl)-carbamic acid 1-methyl-1, 2, 3, 4-tetrahydro-quinolin-6-yl ester
**2p**. (3-bromo-phenyl)-carbamic acid 1-methyl-1, 2,3,4-tetrahydro-quinolin-6-yl ester
**2q.** (4-chloro-3-trifluoromethyl-phenyl)-carbamic acid 1-methyl-1, 2 ,3, 4-tetrahydro-quinolin-6-yl ester
**2r.** (4-bromo-phenyl)-carbamic acid 1-methyl-1, 2,3,4-tetrahydro- quinolin-6-yl ester

The compounds of the invention are prepared by reacting substituted phenols with various isocyanates. In such cases R₁ includes alkyl like hexyl, heptyl, and aryl, and 2-chloro, 3-bromo, 4-bromo, or 4-chloro-3-trifluoromethyl-phenyl; R₂ includes at least methyl or benzyl group. The reaction is carried out using organic and inorganic bases and at least one organic solvent such as ether, tetrahydrofuran (THF), dimethylformamide (DMF), dioxane, dichloromethane or chloroform and at temperature ranging from -10°C to 80°C for a period between half an hour to 100 hours to produce corresponding carbamic acid esters (carbamates).

The synthesis of compounds of formula **2g-r from 2a-f** is achieved by reacting compounds **2a-I** with RX (alkyl or aralkyl halides) wherein R includes at least methyl or benzyl group and X includes at least chloro, bromo or iodio and using a solvent such as DMF, THF and dioxane, in the presence of an organic or inorganic base such as sodium hydride, sodium hydroxide, triethylamine or pyridine at a temperature ranging between -10°C to 37°C for a period of 1 hour to 12 hours in the presence or absence of a catalyst sodium iodide or potassium iodide.

The molar ratio of substituted phenols to isocyanate is 1:1 to 1:1.2. In yet another preferred embodiment of the present invention, the organic solvent is present about 1.0 ml to 10 ml per mmol of the compounds. Preferably, **2a-f** are synthesized from **2m-r** using Pd-C 5-10% in the solvents like ethanol or methanol (15-25 ml for per mmol of the compound) by applying hydrogen pressure 50-60 psi for a period between 4-12 hours at room temperature. Salts of the compounds types **1** and **2** are also included within the scope of the invention.

The invention also relates to a method for enhancing cholinergic activity in conditions linked with hypofunctioning of cholinergic system. Such situations include peripheral as well as central nervous system. Peripheral nervous system disorders in which use of anticholinesterse is indicated are atony of the smooth muscle of the intestinal tract (paralytic ileus), atony of urinary bladder, glaucoma, and myasthenia gravis. In central nervous system the most important area is cognitive behaviour dysfunctions (dementia).

The compound of formula **1** and **2m-r** of the present invention are prepared by one of the following process shown in the following scheme. 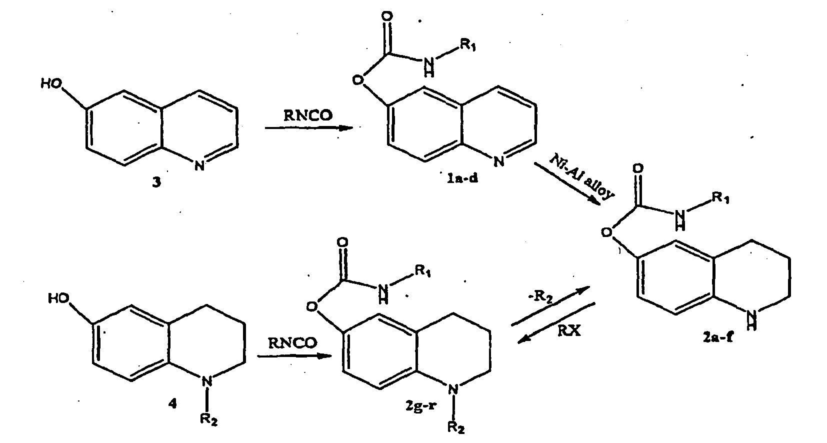 wherein R₁ = alkyl, aryl, 2-chlorophenyl, 3-bromo-phenyl, 4-bromophenyl, 4-chloro-3-trifluoromethyl-phenyl; R₂=H, alkyl, aralkyl.

The phenols **3** and **4** in the above scheme were reacted with various isocyanates to give corresponding carbamates. The carbamates **1a-d** were further reduced with Ni- Al alloy/KOH/ethanol to give the corresponding 1, 2, 3, 4 - tetrahydro derivatives **2a-I.** The N-benzyl derivatives 2m-r were debenzylated using 5% or 10%Pd-C/H₂ in ethanol or methanol as a solvent to give corresponding debenzylated carbamates **2a-f.** These debenzylated carbamates were then N-methylated using MeI to give **2m-r.**

The phenols **3** and **4** in the above scheme, were converted to the corresponding isocyanates using solvents such as tetrahydrofuran, dioxane, dimethylformamide, dichloromethane or chloroform, in the presence of bases like sodium hydride, sodium hydroxide, potassium hydroxide, sodium carbonate, potassium carbonate, pyridine or triethylamine at a temperature ranging from -10°C to 80°C for ½ hour to 100 hours. In methylation of compounds **2a-1** various solvents were used like tetrahydrofuran, dioxane and dimethylformamide at a temperature ranging from 10 to 37°C, for between 3 hours to 48 hours.

The present invention deals with the synthesis and evaluation of various novel substituted carbamic acid quinolinyl esters represented by formulae **1** and **2** for their antiacetylcholinesterase inhibitory activity. These compounds have shown very high antiacetylcholinesterase inhibitory activity when compared with prior art compounds illustrated below.

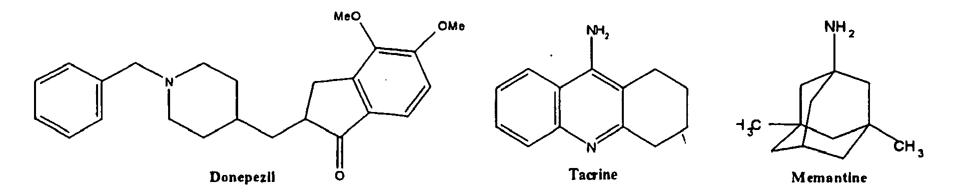

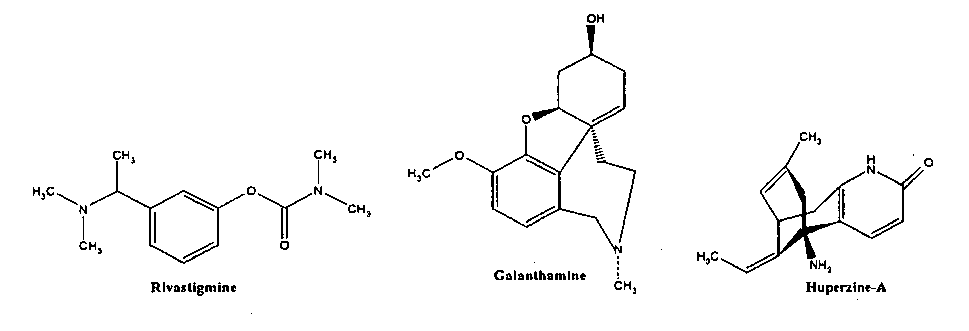

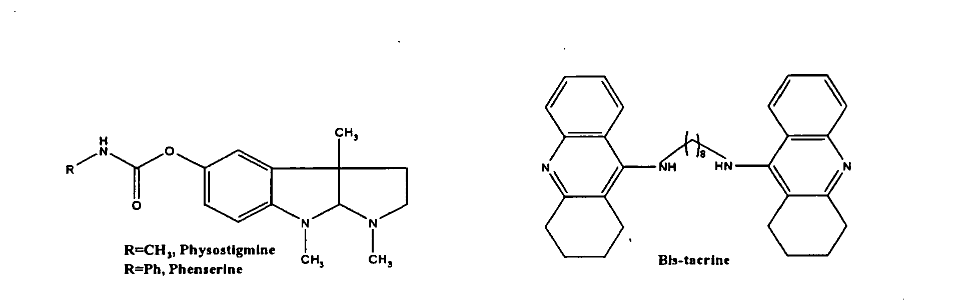

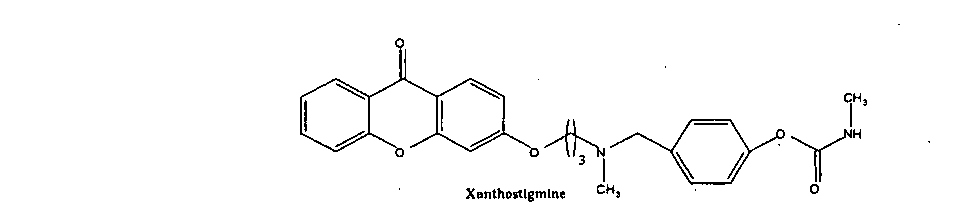

### Pharmacological action of compounds 1 and 2

Pharmacological action of the compounds of the present invention was tested using several compounds prepared in examples presented herein after and tacrine and donepezil as a comparative compound.

### (A) Inhibitive Effect on Acetylcholinesterase Activity

The study was conducted in adult SD male rats (200-250 g). Rats were perfused under mild ether anesthesia through heart with ice cooled normal saline (0.9% NaCl) to reduce blood-borne cholinesterase from brain. After perfusion whole brain was taken out. 10% (w/v) homogenate of brain was prepared first by homogenizing in Ultra-Turrax T25 homogenizer at a speed of 9500 rpm thrice giving intervals for few seconds in between the runs, with sodium phosphate buffer (30 mmol/lit, pH 7.0). Sodium phosphate buffer was taken in a volume half to the final volume required for 10% homogenate. 1% Triton X-100 (1% w/v in 30 mmol/lit. sodium phosphate buffer, pH 7.0) is then added in a volume to make the final volume for 10% homogenate, slowly while stirring the homogenate on ice.

The homogenate was centrifuged at 100,000 x g at 4°C in a Beckman Ultracentrifuge (LE 80) using a fixed angle rotor (80 Ti) for 60 min. Supernatant was collected and stored at 4°C. Aliquots of this supernatant was diluted in the ratio of 1:10 and used as a source of enzyme for the assay.

### Enzyme assay:

Assay of AChE was performed according to method described by Ellman et al., (Ellman, G.E.; Courtney, K.D.; Andersen, V. Jr.; Featherstone, R.M. Biochem. Pharmacol. 1961, 7, 88). Kinetic profile of the enzyme activity was studied spectrophotometrically at 412 nm at an interval of 15 s. Assay for each sample was run in duplicate and each experiment was performed thrice. The specific activity of AChE was calculated by following formula:
AChE activity = ΔE x 1000 x V /1.36 x 10000 x v
ΔE = Extinction change / min
1000 = Conversion factor for µmoles
V = Volume of the total reaction mixture
1.36 x 10000 = Extinction Coefficient
v = volume of the enzyme used

The specific activity of AChE is expressed in µmoles/min/mg of protein.

The test substance (dissolved in DMSO) was incubated with enzyme source in concentration of 100 µg/lml of reaction mixture for 30 min at 37°C prior to obtain kinetic profile of AChE activity. Tacrine (1µmol) was used as standard AChE inhibitor (standard control). The AChE inhibitory activity was calculated on the basis of % decrease change from control values i.e. AChE acivity without incubation with any standard or test drug.

### Protein assay:

Protein was estimated in the brain samples by modified Lowry's method (Wang, C.H.; Smith, R.L Anal. Biochem. 1975, 63, 414). Bovine serum albumin (BSA) was used as standard in the concentration of 1 mg/ml. was estimated in the range of 0.01-0. mg/ml.

**Table 1. Cholinesterase Enzyme Activity (µmol / mg of protein/min) in presence of different concentrations of active drug**

| **(Conc. µm)** | 0.1 | 0.3 | 1 | 3 | 10 |
|---|---|---|---|---|---|
| **Drug** | | | | | |
| Tacrine | 0.01258 | 0.01165 | 0.0027 | 0.0018 | 0.0004 |
| **2i** | 0.01351 | 0.01211 | 0.01071 | 0.007456 | 0.001864 |

**Table 2. % Cholinesterase Enzyme Activity in presence of different concentrations of active drug**

| **(Conc. µm)** | 0.1 | 0.3 | 1 | 3 | 10 |
|---|---|---|---|---|---|
| **Drug** | | | | | |
| Tacrine | 87.09 | 80.64 | 19.35 | 12.90 | 3.22 |
| **2I** | 93.54 | 83.87 | 74.19 | 51.61 | 12.90 |

| | | | | | |
|---|---|---|---|---|---|
| Control AChE activity = 0.014446 (µmol / mg of protein/min) | | | | | |

**Table 3. % Anticholinesterase Enzyme Inhibition in presence of different concentrations of active drug**

| **(Conc. µm)** | 0.1 | 0.3 | 1 | 3 | 10 | IC₅₀ µm |
|---|---|---|---|---|---|---|
| **Drug** | | | | | | |
| Tacrine | 12.90 | 19.35 | 80.64 | 87.09 | 96.77 | 0.8 |
| **2i** | 6.45 | 16.12 | 25.80 | 48.38 | 87.09 | 3.31 |

### (B) Effects on Amnesia Induced by Scopolamine in mice

### Experimental procedures

Single trial passive avoidance is widely used as experimental test to assess learning memory functions in rodents. Scopolamine induced impairment in passive avoidance (*in vivo)* and inhibition of acetylcholinesterase (*in vitro*) in rodents are commonly employed and screening test to predict potential of an acetylcholinesterase inhibitor as cognitive enhancer (anti-dementic) drug (Das, A.; Shanker, G.; Nath C; Pal, R; Singh, S; Singh, H.K; Pharmacol. Biochem. Behav. 2002, 73, 893).
**Passive Avoidance Test (*in vivo*)*****:*** Study was conducted in adult Swiss male mice of 3-4 months (wt. 20-25 g) which were kept in standard housing condition with 12h light and dark cycle. Food and water were available ad libitum. Mice were subjected to single trial passive avoidance test described by Brioni (Brioni, J.D.; Hock, F.J.; McGaugh, J.J. Drug effects on learning and memory in: Vogel, G. H. and Vogel, W.H. (Eds.), Drug Discovery and Evaluation: Pharmacological Assays. Springer Verlag Press , New York,. 1997, pp. 335-336). Passive avoidance test was studied by a computerized shuttle box (Columbus Instruments, Ohio, USA) provided with a software program PACS 30. The shuttle box comprises of two compartments, isolated by an automated door. After exploration period of 30s for acclimatization the animal was subjected to a trial of 270 seconds. Each mouse was placed in the bright compartment and on transfer into the dark compartment it was given an electric shock (0.5 mA for 5 s) through floor grid. Transfer of mice from bright to dark compartment was recorded as transfer latency time (TLT) in seconds. TLT was recorded in control and treated groups (1^{st} Trial, acquisition) and then after 24 hours (2^{nd} Trial, retention). An increase in the TLT on 2^{nd} Trial (retention) as compared to 1^{st} Trial (acquisition) was taken as the criterion for successful learning and memory (cognitive activity).
**Scopolamine induced deficit (Dementia):** Scopolamine a muscarinic antagonist, known to produce impairment in cognitive functions (dementia) in human as well as in experimental animals, was used to produce deficit (no significant increase on 2^{nd} trial) in passive avoidance learning. Scopolamine was administered 5 min prior to 1^{st} trial. Reversal of scopolamine induced deficit i.e. significant increase in 2^{nd} trial by test substance indicates potential anti-dementia activity. Scopolamine was administered 5 min prior to 1^{st} trial.
**Drug administration:** Scopolamine was administered 5 min prior to 1^{st} trial in test group. Each test compound was administered orally in dose of 20µmol/kg (1% aq. suspension in gum acacia) 1 hour prior to 2^{nd} trial in scopolamine treated mice (n=5). Scopolamine control group received 1 ml/kg of vehicle (1% aq. suspension in gum acacia) orally. Scopolamine was administered 5 min prior to 1^{st} trial in test group. Trained control group (n=5) did not receive any drug. Donepezil (10 mg/kg, po, as1% aqueous suspension in gum acacia) was used as standard drug and given 1 hour prior to 2^{nd} trial in scopolamine treated mice.
**Statistical analysis:** Mean values and standard error (S.E.) of mean were calculated for TLT and specific activity of AChE in the different regions of brain samples of each group. The significance of difference between the values of AChE activity and TLT between the groups was determined by one-way ANOVA test that followed by Dunnett's test.

**Table 4: Effect of the compounds on scopolamine induced amnesia in passive avoidance test**

| Group | 1^{st} TRIAL | 2^{nd} TRIAL | % Learning | % Improvement in Learning |
|---|---|---|---|---|
| CONTROL | 66.14 | 248 | 274.9 | 0 |
| SCOPOLAMINE | 110 | 147 | 33.6 | 0 |
| SCO+TACRINE | 73.4 | 241 | 228.3 | 194.7 |
| SCO+DONEPEZIL | 91.4 | 245 | 168 | 134.4 |
| SCO+1b | 99 | 238 | 140.4 | 106.8 |
| SCO+1c | 89.6 | 230 | 156.7 | 123.09 |
| SCO+2c | 97 | 195 | 101 | 67.4 |
| SCO+2h | 92 | 205 | 122.8 | 89.2 |
| SCO+ 2i | 83.5 | 186.4 | 123.2 | 89.6 |
| SCO+2q | 104 | 218 | 109 | 76 |
| SCO+ 2r | 124 | 242 | 95.6 | 62 |

| | | | | |
|---|---|---|---|---|
| % Learning: 2^{nd} Trial - 1^{st} trial/1st trial] x 100; % Improvement in Learning: % Learning in treated gp - % Learning in Scopolamine gp | | | | |

The following examples are given by way of illustration and thereof should not be construed to limit the scope of the present invention.

### Example1. Hexyl-carbamic acid quinolin-6-yl ester (1a)

(a) Mixture of quinolin-6-ol (0.58 g., 4 mmol), dry dioxane (20 ml), hexyl isocyanate (0.698 ml, 4.8 mmol) and dry pyridine (0.2 ml) was stirred at room temperature (32°C) for 48 hours. Reaction mixture was concentrated under vaccum, titurated with water (1ml) and crystallised with ether, to give **1a;** yield: 0.80 g. (73.5%), m.p. 89°C, C₁₆H₂₀N₂O₂; ¹H NMR δ ppm (CDCl₃): 0.91 (bs, 3H), 1.33-1.34 (bs, 6H), 1.56-1.62(m, 2H), 3.24-3.34 (m, 2H), 5.21 (bs, 1H), 7.35-7.42 (m, 1H), 7.48-7.52 (m, 1H), 7.60-7.61 (m, 1H), 8.07-8.12 (m, 2H), 8.86-8.89 (m, 1H); IR νₘₐₓ (KBr) (cm⁻¹): 478, 530,674, 730, 788, 839, 909, 970, 1002, 1040, 1158, 1215, 1258, 1300, 1356, 1462, 1495, 1543, 1709, 1919, 2373, 2861, 2961, 3032, 3286, 3346, 3777, MS: m/z: 273 (M⁺).
(b) Mixture of quinolin-6-ol (0.58 g., 4 mmol), hexyl isocyanate (0.698 ml, 4.8 mmol) and of dry pyridine (0.3 ml) in dry dioxane (10 ml) was heated at 100°C with stirring for 3 hours. Reaction mixture was concentrated under vaccum, the residue was titurated with water (1ml) and crystallised with ether to give **1a;** yield: 0.75 g. (68.93%).
(c) Mixture of quinolin-6-ol (0.58 g., 4 mmol), hexyl isocyanate (0.70 ml, 4.8 mmol) and dry pyridine (0.2ml) in dry dimethylformamide (1ml) was heated at 50°C for 5 hours. Reaction mixture was diluted with water (5ml), extracted with ethyl acetate (2x5 ml) and dried over sodium sulphate. The combined ethyl acetate fractions were concentrated under vaccum and crystallized with ether to give **1a;** yield: 0.7g. (59.7%).
(d) Mixture of quinolin-6-ol (1.16 g., 8 mmol), hexyl isocyanate (1.37 ml, 9.6 mmol) and of dry triethylamine (0.1ml) in dry dimethylformamide (1 ml) was heated at 50°C for 5 hours. Reaction mixture was diluted with water (5 ml), extracted with ethyl acetate (2x5ml), dried over sodium sulphate. The combined ethyl acetate fractions were concentrated under vaccum and crystallized with ether to give **1a;** yield: 1.2 g. (55.14%).

### Example 2: Heptyl-carbamic acid quinolin-6-yl ester (1b)

(a) A mixture of quinolin-6-ol (0.29 g., 2 mmol), heptyl isocyanate (0.386 ml 2.4 mmol) and pyridine (1 ml) in dry tetrahydrofuran (10 ml) was heated at 65°C with stirring for 5 hours. The reaction mixture was cooled and then quenched with water (1 ml), the reaction mixture was concentrated under vaccum and the separated solid was washed with water (2x5ml) and crystallised with ether to give **1b**; yield: 0.25 g. (43.7%), m.p. 78°C, C₁₇H₂₂N₂O₂.; ¹H NMR δ ppm (CDCl₃): 0.88 (bs, 3H), 1.33-1.34 (m, 8H), 1.57-1.62 (m, 2H), 3.25-3.35 (m, 2H) 5.10 (s,1H), 7.36-7.52 (m,1H), 7.47-7.52 (m, 1H), 7.60-7.61 (m, 1H), 8.07-8.12 (m, 2H), 8.86-8.88 (m, 1H); IR νₘₐₓ (KBr) (cm⁻¹): 478, 648, 731, 771, 838, 910, 977, 1024, 1157, 1215, 1363, 1464, 1498, 1532, 1600, 1719, 2371, 2861, 2944, 3022, 3359, 3762; MS: m/z: 287 (M⁺).
(b) A mixture of quinolin-6-ol (0.29 g., 2 mmol), heptyl isocyanate (0.39 ml, 2.4 mmol) and pyridine (2 ml) in dry tetrahydrofuran (10 ml) was stirred at room temperature (21°C) for 40 hours, the reaction mixture was concentrated under vaccum and the residue was washed with water (2x5ml) and crystallized with ether to give **1b;** yield: 0.30 g. (52.4%).
(c) A mixture of quinolin-6-ol (0.58 g., 4 mmol), heptyl isocyanate (0.77 ml, 4.8 mmol), potassium carbonate (0.56 g., 4 mmol) and sodium iodide (0.60 g., 4mmol) in dry dimethylformamide (2 ml) was stirred at 50°C for 12 hours. The reaction mixture was diluted with water (5ml), extracted with ethyl acetate (2x5ml) and chromatographed on silica gel with chloroform as an eluant to give **(1b);** yield: 0.50 g. (43.7%).

### Example 3. (2-Chloro-phenyl)-carbamic acid quinolin-6-yl ester (1c)

(a) A mixture of quinolin-6-ol (0.29 g., 2 mmol), 2-chloro-phenyl isocyanate (0.33 ml, 2.4 mmol) and pyridine (0.3 ml) in dry tetrahydrofuran (5 ml) was heated at 65°C with stirring for 5 hours. The reaction mixture was cooled, quenched with water (1 ml) and concentrated under vaccum. The separated solid was washed with water (2x5ml) and crystallised with methanol to give **1c;** yield: 0.30 g. (50.2%), m.p. 228°C, C₁₆H₁₁ClN₂O₂. ¹H NMR δ ppm (pyridine d₅): 6.96-7.57(m, 9H), 8.62-8.71(m, 1H), 9.47(m, 1H); IR νₘₐₓ (KBr) (cm⁻¹): 903, 943, 1040, 1230, 1291, 1353, 1437, 1474, 1552, 1592, 1646, 2373, 3289, 3759.
(b) A mixture of quinolin-6-ol (0.145 g., 1 mmol), 2-chloro-phenyl isocyanate (0.164 ml, 1.2 mmol), potassium carbonate (0.14 g., 1 mmol), potassium iodide (0.16, 1 mmol) g. in dry dimethylformamide (2 ml) was heated at 80°C for 5 hours. The reaction mixture was diluted with water (5ml), extracted with ethyl acetate (2x5ml) and concentrated under vaccum. The residue was washed with methanol (2x2ml) to give 1c; yield: 0.16 g. (53.6%).
(c) Mixture of quinolin-6-ol (0.29g., 2mmol), 2-chloro-phenyl isocyanate (0.33 g., 2.4 mmol), potassium carbonate (0.28 g., 2 mmol) and potassium iodide (0.32 g., 2 mmol) in dry dioxane (10 ml) was heated at 100°C with stirring for 4 hours. Reaction mixture was concentrated under vaccum, diluted with water, extracted with ethyl acetate (2x5ml) and concentrated under vaccum. Residue was washed with methanol (2x3ml) to give **1c;** yield: 0.30g. (50.2%).

### Example 4. (3-bromo-phenyl)-carbamic acid quinolin-6-yl ester (1d)

(a) A solution of quinolin-6-ol (0.29 g., 2 mmol) in dry dimethylformamide (3 ml) was added to a stirred suspension of sodium hydride (0.048 g., 2 mmol) in dry dimethylformamide (2 ml) at -10°C during 5 min. The reaction mixture was stirred for ½ hours. Then 3-bromo-phenyl isocyanate (0.475 g., 2.4 mmol) was added to the stirring reaction mixture. Stirring was continued for additional 1 hr. during which the temperature was allowed to rise to room temperature (21°C). The reaction mixture was quenched with water (0.2 ml), diluted with water (2ml), extracted with ethyl acetate (2x5 ml) and dried over sodium sulphate. The combined fractions of ethyl acetate were concentrated under vaccum. The residue was washed with methanol (2x2ml) to give **1d;** yield: 0.35g. (51.0%), m.p. 260°C, C₁₆H₁₁BrN₂O₂; ¹H NMR δ ppm (pyridine d₅): 7.09-7.58(m, 9H), 8.28(bs, 2H), 9.58(bs, 1H); IR νₘₐₓ (KBr) (cm⁻¹): 522, 645, 743, 782, 877, 928, 992, 1069, 1227, 1286, 1406, 1470, 1581, 1637, 1875, 1946,2374,3290,3753,3872,3952.
(b) A mixture of quinolin-6-ol (0.29 g., 2 mmol), 3-bromo-phenyl isocyanate (0.47 g., 2.4 mmol), potassium carbonate 0.28 g (0.28 g 2 mmol) and potassium iodide (0.32 g.) in dry dimethylformamide (2 ml) was heated at 80°C for 20 hours. The reaction mixture was diluted with water (5ml), extracted with ethyl acetate (2x5ml) and dried over sodium sulphate. The combined ethyl acetate fractions were concentrated under vaccum. The residue was washed with methanol (2x3ml) to give **1d**; yield: 0.34 g. (49.5%).

### Example 5. Hexyl-carbamic acid 1, 2, 3, 4-tetrahydro-quinolin-6-yl ester (2a)

A mixture of hexyl-carbamic acid 1-benzyl-1, 2, 3, 4-tetrahydro-quinolin-6-yl ester (0.732 g., 2 mmol) and 5% Pd-C (0.08 g.) in absolute ethanol (20 ml) was shaken in a par apparatus at room temperature (38°C) under 50 psi pressure of hydrogen for 4 hours. Pd-C was then discarded through filtration. The reaction mixture was concentrated under vaccum and the separated solid was washed with chloroform (2x5ml) to give **2a;** yield: 0.60 g. (64.1%), m.p.: 100°C, C₁₆H₂₄N₂O₂; ¹H NMR δ ppm (CDCl₃): 0.88(bs, 3H), 1.25-1.30 (m, 5H), 1.51-1.58 (m, 3H), 1.85-1.97 (m, 3H), 2.74 (t, 2H), 3.16-3.29(m,4H), 3.6( bs, 1H), 4.87 (bs,1H), 6.39-6.4 (m, 1H), 6.68-6.71 (m, 2H); IR νₘₐₓ(KBr) (cm⁻¹): 770, 816, 886, 956, 1006, 1101, 1152, 1221, 1243, 1311, 1354, 1505, 1626, 1702, 1848, 2372, 2856, 2929, 3392, 3760, 3809, 7874; MS: m/z: 277 (M⁺).

### Example 6. Heptyl-carbamic acid 1, 2, 3, 4-tetrahydro-quinolin-6-yl ester (2b)

(a) A nitrogen flushed mixture of heptyl-carbamic acid 1-benzyl-1, 2, 3, 4-tetrahydro-quinolin-6-yl ester (0.378 g., 1mmol) and 10% Pd-C (0.02 g.) in absolute ethanol (20 ml) was shaken in a par apparatus at room temperature (38°C) under 55 psi pressure of hydrogen for 4 hours. Pd-C was then discarded through filtration. The reaction mixture was concentrated under reduced pressure and the residue was chromatographed with methanol: dichloromethane (1:99) to give **2b;** yield: 0.25 g. (84.6%), m.p.: 67°C, C₁₇H₂₆N₂O₂; ¹H NMR δ ppm (CDCl₃): 0.88 (bs,3H), 1.20-1.30 (m, 7H), 1.56 (bs, 3H), 1.88-1.93 (m,2H), 2.74 (t, 2H), 3.23-3.28 (m,4H), 3.66-3.76 (m, 1H), 4.89 (bs, 1H), 6.40-6.44 (m, 1H), 6.68-6.71 (m, 2H); IR νₘₐₓ (KBr) (cm⁻¹): 561 , 665, 768, 815, 887, 976, 1149, 1223, 1308, 1353, 1506, 1621, 1699, 2362, 2857, 2930, 3763.
(b) Ni-Al alloy (0.098 g.) was added portionwise during 1 min. to stirring mixture of heptyl-carbamic acid quinolin-6-yl ester (0.145 g.), 10% potassium hydroxide in water (3.54 ml) and ethanol (10 ml) at 0-4°C. The reaction mixture was continued to stir for additional 2.5 hours while the temperature of the reaction mixture was maintained at 50°C. The reaction mixture was filtered, Ni-Al alloy was discarded through filtration, and the filtrate was concentrated under vaccum. The residue so obtained was crystallized with methanol (or acetone) to give **2b;** yield: 0.10g. (67.7%).

### Example 7. (3-Bromo-phenyl)-carbamic acid 1,2,3,4-tetrahydro-quinolin-6-yl ester (2c)

A nitrogen flushed mixture of 3-bromo-phenyl -carbamic acid 1-benzyl-1, 2, 3, 4-tetrahydro-quinolin-6-yl ester (0.218 g., ½ mmol) and 5% Pd-C (0.02 g.) in absolute ethanol (25 ml) was shaken in a par apparatus at room temperature (38°C) under 50 psi pressure of hydrogen for 4 hours. Pd-C was then discarded through filtration. The reaction mixture was concentrated under reduced pressure and the residue was washed with dichloromethane (2x3ml) to give **2c;** yield: 0.16 g. (92.4. %), m.p.: 198°C, C₁₆H₁₅BrN₂O₂; ¹H NMR δ ppm (CDCl₃+DMSO-d₆): 1.73-1.81 (m, 2H), 2.55 (t, 2H), 3.06(t, 2H), 6.67-6.98 (m, 4H), 6.90-7.20 (m, 2H), 7.36 (bs, 1H), 9.39 (bs, 1H); IR νₘₐₓ (KBr) (cm⁻¹): 509, 694, 758, 815, 886, 1012, 1084, 1149, 1216, 1319, 1352, 1442, 1504, 1549, 1600, 1710, 2365, 2479, 2727, 2827, 2927, 3420, 3780.

### Example 8: (2-Chloro-phenyl)-carbamic acid 1, 2, 3, 4-tetrahydro-quinolin-6-yl ester (2d) .

A nitrogen flushed mixture of 3-chloro-phenyl -carbamic acid 1-benzyl-1, 2, 3, 4-tetrahydro-quinolin-6-yl ester (0.196 g., ½ mmol) and 5% Pd-C (0.02 g.) in absolute ethanol (25 ml) was shaken in a par apparatus at room temperature (40°C) under 50 psi pressure of hydrogen for 5 hours. Then Pd-C was discarded through filtration. The reaction mixture was concentrated under reduced pressure and the residue was washed with dichloromethane (2x3ml) to give **2d;** yield: 0.11 g. (72.8. %), m.p.: 201°C, C₁₆H₁₅ClN₂O₂; ¹H NMR δ ppm (CD₃OD): 2.00-2.12 (m, 2H), 2.88 (t, 2H), 3.44 (t, 2H), 6.97-7.41 (m, 7H); IR νₘₐₓ(KBr) (cm⁻¹): 509, 609, 699, 753, 814, 857, 887, 9297, 1006, 1064, 1207, 1316, 1351, 1386, 1441, 1501, 1600, 1748, 1960, 2484, 2645, 2725, 2830, 2889, 3134, 3251, 3416.

### Example 9: (4-Chloro-3-trifiuoromethyl-phenyl)-carbamic acid 1, 2, 3, 4-tetrahydro-quinolin-6-yl ester (2e)

A nitrogen flushed mixture of 4-chloro-3-trifluoromethyl-phenyl-carbamic acid 1-benzyl-1, 2, 3, 4-tetrahydro-quinolin-6-yl ester (0.23 g., ½ mmol) and 5% Pd-C (0.02 g.) in absolute ethanol (20 ml) was shaken in a par apparatus at room temperature (38°C) under 50 psi pressure of hydrogen for 5 hours. Pd-C was then discarded through filtration. The reaction mixture was concentrated under reduced pressure and the residue was washed dichloromethane (2x3ml) to give **2e**; yield: 0.16 g. (86.4. %), m.p.: 150°C, C₁₇H₁₄ClF₃N₂O₂; ¹H NMR δ ppm (DMSO-d₆): 1.89-1.95(m, 2H), 2.76(t, 2H), 3.28 (t, 2H), 6.44-6.49 (m,1H), 6.72-6.75 (m, 2H), 7.37- 8.0 (m, 2H),10.14 (bs, 1H); IR νₘₐₓ (KBr) (cm⁻¹): 661, 698, 769, 796, 819, 892, 944, 1023, 1072, 1147, 1217, 1290, 1347, 1384, 1424, 1450, 1506, 1599, 1707, 1740, 2364, 2489, 2837, 2950, 3359, 3773, 3888.

### Example 10: (4-bromo-phenyl)-carbamic acid 1,2,3,4-tetrahydro-quinolin-6-yl ester (2f)

Nitrogen flushed mixture of 4-bromo-phenyl-carbamic acid 1-benzyl-1,2,3,4-tetrahydro-quinolin-6-yl ester (0.655g., 1.5mmol) and 5% Pd-C (0.06 g.) in absolute ethanol (20 ml) was shaken in a par apparatus at room temperature (40°C) under 50 psi pressure of hydrogen for 5 hours. Then Pd-C was discarded through filtration. The reaction mixture was concentrated under reduced pressure and the residue was washed with dichloromethane (2x3ml) to give 2f; yield: 0.48 g. (92.3. %), m.p.: 198°C, C₁₆H₁₅BrN₂O₂; ¹H NMR δ ppm (DMSO): 1.82-2.00 (bs, 2H), 2.76 (t, 2H), 3.27 (t, 2H), 7.01-7.08 (m, 3H), 7.25-7.39 (m, 4H), 10.14(s, 1H); IR νₘₐₓ (KBr) (cm⁻¹): 506, 608, 690, 749, 814, 884, 927, 1005, 1211, 1265, 1319, 1354, 1401, 1440, 1501, 1550, 1601, 1748, 1938, 2370, 2779, 2725, 2767, 2823, 2922, 3262, 3420, 3774.

### Example 11: (a) Heptyl-carbamic acid 1-benzyl-1,2,3,4-tetrahydro-quinolin-6-yl ester (2g)

Mixture of 1-benzyl-1, 2, 3, 4-tetrahydro-quinolin-6-ol (0.239 g., 1 mmol), heptyl isocyanate (0.169 g., 1.2 mmol) and pyridine (1 ml) in dry tetrahydrofuran (10 ml), was heated with stirring at 65°C for 48 hours. Reaction mixture was quenched with water (1 ml) and concentrated under vaccum. The separated solid was washed with water (2x5 ml) and crystallised with ether to give **(2g)** as oil; yield: 0.200 g. (52.6%), C₂₄H₃₂N₂O₂; ¹H NMR δ ppm (CDCl₃): 0.86 (bs, 3H), 1.20-1.29 (m, 6H), 1.55-1.58(m, 4H), 1.96-2.06 (m, 2H), 2.79 (t, 2H), 3.17-3.36 (m, 4H), 4.44 (st, 2H), 4.87 (bs, 1H), 6.39-6.44 (m,1H), 6.71-6.75 (m, 2H), 7.25-7.34 (m, 5H); IR νₘₐₓ (Neat) (cm⁻¹): 757, 902, 1.70, 1160, 1199, 1228, 1348, 1458, 1502, 1618, 1718, 2362, 2857, 2928, 3028, 3342, 3854; MS: m/z=380 (M⁺).
(b) A mixture of 1-benzyl-1, 2, 3, 4-tetrahydro-quinolin-6-ol (0.239 g., 1 mmol), heptyl isocyanate (0.169ml), 1.2 mmol) and pyridine (2 ml) in dry dichloromethane (10 ml) was heated with stirring at 45°C with stirring for 100 hours. The reaction mixture was cooled, washed with water (3x5ml) and dried over sodium sulphate. The reaction mixture was concentrated under vaccum. The residue so obtained was chromatographed on silica gel using dichloromethane: hexane (25:75) as eluant to give **2g**; yield: 0.090 g. (39.03%).

### Example 12: Hexyl-carbamic acid 1-benzyl-1, 2, 3, 4-tetrahydro-quinolin-6-yl ester (2h)

Mixture of 1-benzyl-1,2,3,4-tetrahydro-quinolin-6-ol (0.239g., 1mmol), hexyl isocyanate (0.152g., 1.2mmol) and pyridine (0.5ml) in dry THF (10ml) was heated with stirring at 65°C for 72 hours. Reaction mixture was cooled, quenched with water (1ml) and concentrated under vaccum. Separated solid was washed with water (2x3ml) and crystallised with ether to give 2h; yield: 0.30g. (81.9%), m.p.>335, C₂₃H₃₀N₂O₂; ¹H NMR δ ppm (CDCl₃): 0.89 (bs, 3H), 0.90-1.29 (m, 4H), 1.50-1.56(m, 4H), 1.98-2.15 (m, 2H), 2.79 (t, 2H), 3.17-3.36 (m, 4H), 4.44 (s, 2H), 4.88 (bs, 1H), 6.42 (d,1H), 6.67-6.75 (m, 2H), 7.22-7.35(m, 5H); IR νₘₐₓ (KBr) (cm⁻¹): 669, 760, 1026, 1217, 1348, 1501, 1615, 1728, 2402, 2857, 3018, 3450.
(b) Mixture of 1-benzyl-1,2,3,4-tetrahydro-quinolin-6-ol (0.239 g., 1mmol), hexyl isocyanate (0.174 ml, 1.2 mmol) and triethylamine (1 ml) in dry chloroform (10ml) was heated with stirring for 48 hours. The reaction mixture was cooled, washed with water (2x5 ml), dried over sodium sulphate and concentrated under vaccum. The residue so obtained was chromatographed with 20:80 chloroform:hexane as eluant to give **2h**; yield: 0.25 g. (68.3%).

### Example 13: (2-Chloro-phenyl)-carbamic acid 1-benzyl-1, 2, 3, 4-tetrahydro-quinolin-6-yl ester (2i)

(a) A solution of 1-benzyl-1, 2, 3, 4-tetrahydro-quinolin-6-ol (0.239 g., 1 mmol) in dry tetrahydrofuran (5 ml) was added to a stirred suspension of sodium hydride (0.024 g., 1 mmol) in dry tetrahydrofuran (5 ml) at -10°C during 5 min. The reaction mixture was stirred for 20 min. Then 2-chloro-phenyl isocyanate was added (0.184 g., 1.2 mmol) was added to the stirring reaction mixture. Stirring was continued for additional 2.5 hours during which the temperature was allowed to rise to room temperature (34°C). The reaction mixture was quenched with water (0.2 ml), concentrated under vaccum, diluted with water (5 ml), extracted with chloroform (2x5ml) and dried over sodium sulphate. The combined fractions of chloroform were concentrated under vaccum. The residue was chromatographed on silica gel using chloroform: hexane (20:80) as eluant to give 2i; yield: 0.20g. (50.9%), m.p. 130°C, C₂₃H₂₁ClN₂O₂; ¹H NMR δ ppm (CDCl₃): 1.90-2.02 (m, 2H), 2.82 (t, 2H), 3.30-3.35(m, 2H), 4.46 (s, 2H), 6.43-6.48 (m, 1H), 6.74-6.82 (m, 2H), 7.20-7.25 (m, 5H), 7.44-7.46 (m, 4H); IR *ν*ₘₐₓ (KBr) (cm⁻¹): 534, 754, 807, 882, 1018, 1059, 1193, 1245, 1303, 1352, 1432, 1506, 1597, 1718, 1749, 2371, 2830, 2922, 3418, 3760.
(b) A solution of 1-benzyl-1, 2, 3, 4-tetrahydro-quinolin-6-ol (0.239 g., 1 mmol) in dry ether (10 ml) was added to a stirred suspension of sodium hydride (0.024 g., 1 mmol) in dry ether (15 ml) at -10°C during 10 min. The reaction mixture was stirred for 20 min. Then 2-chloro-phenyl isocyanate (0.184 g., 1.2 mmol) was added to the stirring reaction mixture. Stirring was continued for additional 2 hours during which the temperature was allowed to rise to room temperature (35°C). The reaction mixture was quenched with water (0.2 ml), concentrated under vaccum, diluted with water (5 ml), extracted with chloroform (2x5ml) and dried over sodium sulphate. The combined fractions of chloroform were concentrated under vaccum. The residue was chromatographed on silica gel using chloroform: hexane (20:80) as eluant to give **2i**; yield: 0.25g. (63.7%).

### Example 14: (3-bromo-phenyl)-carbamic acid 1-benzyl-1, 2, 3, 4-tetrahydro-quinolin-6-yl ester (2j)

A solution of 1-benzyl-1, 2, 3, 4-tetrahydro-quinolin-6-ol (0.239 g., 1 mmol) in dry tetrahydrofuran (5 ml) was added to a stirred suspension of sodium hydride (0.024 g., 1mmol) in dry tetrahydrofuran (5 ml) at -10°C during 5 min. The reaction mixture was stirred for 20 min. Then 3-bromo-phenyl isocyanate (0.237 g., 1.2 mmol) was added to the stirring reaction mixture. Stirring was continued for additional 2.5 hours during which the temperature was allowed to rise to room temperature (34°C). The reaction mixture was quenched with water (0.2 ml), concentrated under vaccum, diluted with water (5 ml), extracted with chloroform (2x5ml) and dried over sodium sulphate. The combined fractions of chloroform were concentrated under vaccum. The residue was chromatographed on silica gel using chloroform: hexane (20:80) as eluant to give **2j;** yield: 0.25g. (57.2%), m.p. 117°C, C₂₃H₂₁BrN₂O₂; ¹H NMR δ ppm (CDCl₃): 2.00-2.20 (m, 2H), 2.81 (t, 2H), 3.36 (t, 2H), 4.46 (s, 2H), 6.46 (d, 1H), 6.79-6.81 (m, 2H), 7.19-7.31 (m, 8H), 7.69 (s, 1H); IR νₘₐₓ (KBr) (cm ¹): 621, 670, 759, 885, 930, 1021, 1117, 1215, 1348, 1420, 1505, 1594, 1743, 2369, 2855, 2928, 3021, 3428; MS: m/z=437 (M⁺).

### Example 15. (4-Chloro-3-trifluoromethyl-phenyl)-carbamic acid 1-benzyl-1, 2, 3, 4-tetrahydro-quinolin-6-yl ester (2k)

(a) A solution of 1-benzyl-1, 2, 3, 4-tetrahydro-quinolin-6-ol (0.239 g., 1 mmol) in dry tetrahydrofuran (5 ml) was added to a stirred suspension of sodium hydride (0.024 g., 1 mmol) in dry tetrahydrofuran (5 ml) at -10°C during 5 min. The reaction mixture was stirred for 20 min. Then 4-chloro-3-trifluoromethyl-phenyl isocyanate (0.265 g., 1 mmol) was added to the stirring reaction mixture. Stirring was continued for additional 2.5 hours during which the temperature was allowed to rise to room temperature (34°C). The reaction mixture was quenched with water (0.2 ml), concentrated under vaccum, diluted with water (5 ml), extracted with chloroform (2x5ml) and dried over sodium sulphate. The combined fractions of chloroform were concentrated under vaccum. The residue was chromatographed on silica gel using chloroform: hexane (20:80) as eluant to give **2k;** yield: 0.25g. (54.2%), m.p. 151°C, C₂₄H₂₀ClF₃N₂O₂; ¹H NMR δ ppm (CDCl₃): 2.00 (bs, 2H), 2.79 (t, 2H), 3.36 (t, 2H), 4.46 (s, 2H), 6.42-6.46 (m, 1H), 6.70-6.79 (m, 2H), 6.96 (s, 1H), 7.23-7.31 (m, 5H), 7.75-7.82 (m, 2H); IR νₘₐₓ(KBr) (cm⁻¹): 555, 701, 898, 1027, 1218, 1362, 1509, 1635, 1721, 2375, 2658, 2957, 3028, 3449, 3756; MS: m/z=461 (M⁺).
(b) A mixture of 1-benzyl-1, 2, 3, 4-tetrahydro-quinolin-6-ol (0.24 g., 1 mmol), 4-chloro-3-trifluoromethyl-phenyl isocyanate (0.265 g., 1 mmol), pulverized sodium hydroxide (0.044 g., 1 mmol) in dry tetrahydrofuran (20 ml) was stirred for 14 hours at room temperature (35°C). The reaction mixture was concentrated under vaccum, diluted with water, extracted with chloroform and concentrated under vaccum. The residue was then chromatographed on silica gel using chloroform: hexane (30:70) as eluant to give **2k;** yield: 0.20 g. (43.4%).

### Example 16. (4-bromo-phenyl)-carbamic acid 1-benzyl-1, 2, 3, 4-tetrahydro-quinolin-6-yl ester (2l)

(a) A solution of 1-benzyl-1, 2, 3, 4-tetrahydro-quinolin-6=ol (0.239 g., 1 mmol) in dry tetrahydrofuran (5 ml) was added to a stirred suspension of sodium hydride (0.024 g., 1 mmol) in dry tetrahydrofuran (5 ml) at -10°C during 5 min. The reaction mixture was stirred for 20 min. Then 4-bromo-phenyl isocyanate (0.237 g., 1.2 mmol)) was added to the stirring reaction mixture. Stirring was continued for additional 2.5 hours during which the temperature was allowed to rise to room temperature (35°C). The reaction mixture was quenched with water (0.2 ml), concentrated under vaccum, diluted with water (5 ml), extracted with chloroform (2x5 ml) and dried over sodium sulphate. The combined fractions of chloroform were concentrated under vacuum. The residue was chromatographed on silica gel using (20:80) chloroform: hexane as eluant to give **2l;** yield: 0.26g. (59.4%); m.p. 170°C; C₂₃H₂₁BrN₂O₂; ¹H NMR δ ppm (CDCl₃): 2.00 (bs, 2H), 2.81 (t, 2H), 3.36 (t, 2H), 4.46 (s, 2H), 6.44 (d, 1H), 6.79-6.81 (m, 2H), 7.26-7.45 (m, 9H); IR νₘₐₓ(KBr) (cm⁻¹): 501, 694, 765, 813, 1010, 1070, 1219, 1352, 1505, 1599, 1716, 2225, 2375, 2830, 2934, 3330, 3757; MS: m/z=437 (M⁺).
(b) A mixture of 1-benzyl-1, 2, 3, 4-tetrahydro-quinolin-6-ol (0.239 g., 1 mmol), 4-chloro-3-trifluoromethyl-phenyl isocyanate (0.237 g., 1 mmol), pulverized potassium hydroxide (0.057 g., 1 mmol) in dry dimethylformamide (2 ml) was stirred for 12 hours at room temperature (35°C). The reaction mixture was diluted with water (5 ml), extracted with ethyl acetate (3x5ml), dried over sodium sulphate and the combined ethyl acetate fractions were concentrated under vaccum. The residue was then chromatographed on silica gel using dichloromethane: hexane (20:80) as eluant to give **2l**; yield: 0.18 g. (41.2%).

### Example 17. Hexyl-carbamic acid 1-methyl-1, 2, 3, 4-tetrahydro-quinolin-6-yl ester (2m)

(a) A solution of 1-methyl-1, 2, 3, 4-tetrahydro-quinolin-6-ol (0.244 g., 1 mmol) in dry tetrahydrofuran (5 ml) was added to a stirred suspension of sodium hydride (0.024 g., 1. mmol) in dry tetrahydrofuran (5 ml) at -10°C during 5 min. The reaction mixture was stirred for 20 min. Then hexyl isocyanate (0.26 g., 1 mmol) was added to the stirring reaction mixture. Stirring was continued for additional 2.5 hours during which the temperature was allowed to rise to room temperature (34°C). The reaction mixture was quenched with water (0.2 ml), concentrated under vaccum, diluted with water (5 ml), extracted with chloroform (2x5ml) and dried over sodium sulphate. The combined fractions of chloroform were concentrated under vaccum. The residue was chromatographed on silica gel using chloroform: hexane (20:80) as eluant to give **2m;** yield: 0.25g. (57.9%), m.p. 50°C, C₁₇H₂₆N₂O₂; ¹H NMR δ ppm (CDCl₃): 0.88 (bs, 3H), 1.26-1.54 (m, 8H), 1.96-1.99 (m, 2H), 2.71 (t, 2H), 2.85(s, 3H), 3.14-3.25(m, 4H), 4.90 (bs, 1H), 6.54 (d, 1H), 6.71-6.87 (m, 2H); IR νₘₐₓ(KBr) (cm⁻¹): 656, 757, 895, 1160, 1226, 1319, 1387, 1502, 1721, 2932, 1914, 2370, 2863, 3339, 3773; MS: m/z: 391 (M⁺).
(b) A solution of hexyl-carbamic acid-1, 2, 3, 4-tetrahydro-quinolin-6-yl ester (0.276 g., 1 mmol) in dry tetrahydrofuran (5 ml) was added to a stirred suspension of sodium hydride (0.024 g., 1mmol) in dry tetrahydrofuran (5 ml) at -10°C during 5 min. The reaction mixture was stirred for 20 min. Then methyl iodide (0.224 ml, 3.6 mmol) was added to the stirring reaction mixture. Stirring was continued for additional 1.25 hours during which the temperature was allowed to rise to room temperature (34°C). The reaction mixture was quenched with water (0.2 ml), concentrated under vaccum, diluted with water (5 ml), extracted with chloroform (2x5ml) and dried over sodium sulphate. The combined fractions of chloroform were concentrated under vaccum. The residue was chromatographed on silica gel using chloroform: hexane (30:70) as eluant to give **2m;** yield: 0.15 g. (51.7%).

### Example 18. Heptyl-carbamic acid 1-methyl-1,2,3,4-tetrahydro-quinolin-6-yl ester (2n)

(a) A solution of 1-methyl-1, 2, 3, 4-tetrahydro-quinolin-6-ol (0.490 g., 3 mmol) in dry dimethylformamide (5 ml) was added to a stirred suspension of sodium hydride (0.072 g., 3 mmol) in dry dimethylformamide (5 ml) at -10°C during 5 min. The reaction mixture was stirred for 20 min. Then heptyl isocyanate (0.58 g., 3 mmol) was added to the stirring reaction mixture. Stirring was continued for additional 2.5 hours during which the temperature was allowed to rise to room temperature (37°C). The reaction mixture was quenched with water (0.2 ml), diluted with water (5 ml), extracted with ethyl acetate (2x5ml) and dried over sodium sulphate. The combined fractions of ethyl acetate were concentrated under vaccum. The residue was chromatographed on silica gel using chloroform: hexane (20:80) as eluant to give **2n;** yield: 0.60 g. (65.6%), m.p. 60°C, C₁₈H₂₈N₂O₂; ¹H NMR δ ppm (CDCl₃): 0.88 (bs, 3H), 1.30-1.56 (m, 10H), 1.95-2.05 (m, 2H), 2.68 (t, 2H), 2.86 (s, 3H), 3.15-3.26(m, 4H), 6.52 (d, 1H), 6.70-6.86 (m, 2H); IR νₘₐₓ (KBr) (cm⁻¹): 758, 899, 1160, 1226, 1318, 1390, 1503, 1621, 1718, 2861, 2930, 3348, 3759; MS: m/z: 305 (M⁺).
(b) A mixture of heptyl-carbamic acid-1, 2, 3, 4-tetrahydro-quinolin-6-yl ester (0.58g., 2 mmol), methyl iodide (0.224 ml, 3.6 mmol) and pulverized potassium hydroxide (0.16 g., 2 mmol) in dry tetrahydrofuran (10 ml) was stirred for 5 hours at room temperature (36°C). The reaction mixture was concentrated under vaccum, diluted with water (5 ml), extracted with ether (3x5ml), dried over sodium sulphate and the combined ether fractions were concentrated under vaccum. The residue was then chromatographed on deactivated silica gel (water: silica gel =12:88; v/w) using dichloromethane: hexane (20:80) as eluant to give **2n**; yield: 0.36 g. (59.2%).

### Example 19. (2-Chloro-phenyl)-carbamic acid 1-methyl-1, 2, 3, 4-tetrahydro-quinolin-6-yl ester (2o)

(a) A solution of 1-methyl-1, 2, 3, 4-tetrahydro-quinolin-6-ol (0.326 g., 2 mmol) in dry dimethylformamide (5 ml) was added to a stirred suspension of sodium hydride (0.048 g., 2 mmol) in dry dimethylformamide (5 ml) at -10°C during 5 min. The reaction mixture was stirred for 30 min. Then 2-chloro-phenyl isocyanate (0.329 g., 2mmol) was added to the stirring reaction mixture. Stirring was continued for additional 2.5 hours during which the temperature was allowed to rise to room temperature (35°C). The reaction mixture was quenched with water (0.2 ml), diluted with water (5 ml), extracted with ethyl acetate (2x5 ml) and dried over sodium sulphate. The combined fractions of ethyl acetate were concentrated under vaccum. The residue was chromatographed on silica gel using chloroform: hexane (20:80) as eluant to give **2o;** yield: 0.50 g. (78.9%), m.p. 120°C, C₁₇H₁₇ClN₂O2; ¹H NMR δ ppm (CDCl₃): 1.95-2.01 (m, 2H), 2.77 (t, 2H), 2.88 (s, 3H), 3.21 (t, 2H), 6.54-6.58 (m, 1H), 6.81-7.05(m, 3H), 7.23-7.44 (m, 2H), 8.18-8.22 (m, 1H);
   IR νₘₐₓ(KBr) (cm⁻¹): 563, 670, 758, 1017, 1201, 1305, 1432, 1507, 1596, 1746, 2375, 2940, 3020, 3282, 3413, 3686, 3763.
(b) A solution of 1 of 2-chloro-phenyl -carbamic acid-1, 2, 3, 4-tetrahydro-quinolin-6-yl ester (0.302 g., 1.5 mmol) in dry dioxane (5 ml) was added to a stirred suspension of sodium hydride (0.036 g., 1.5 mmol) in dry dioxane (5 ml) at -10°C during 5 min. The reaction mixture was stirred for 20 min. Then methyl iodide (0.122 ml, 1.8 mmol) was added to the stirring reaction mixture. Stirring was continued for additional 1.5 hours during which the temperature was allowed to rise to room temperature (34°C). The reaction mixture was quenched with water (0.2 ml), concentrated under vaccum, diluted with water (5 ml), extracted with chloroform (2x5ml) and dried over sodium sulphate. The combined fractions of chloroform were concentrated under vaccum. The residue was chromatographed on silica gel using 30:70 chloroform: hexane (30:70) as eluant to give **2o**; yield: 0.50 g. (47.5%).

### Example 20. (3-bromo-phenyl)-carbamic acid 1-methyl-1, 2, 3, 4-tetrahydro-quinolin-6-yl ester (2p)

(a) A solution of 1-methyl-1, 2, 3, 4-tetrahydro-quinolin-6-ol (0.326 g., 2 mmol) in dry tetrahydrofuran (5 ml) was added to a stirred suspension of sodium hydride (0.048 g., 2 mmol) in dry tetrahydrofuran (5 ml) at -10°C during 5 min. The reaction mixture was stirred for 30 min. Then 2- bromo -phenyl isocyanate (0.30 g., 2.4 mmol) was added to the stirring reaction mixture. Stirring was continued for additional 1.5 hours during which the temperature was allowed to rise to room temperature (35°C). The reaction mixture was quenched with water (0.2 ml), concentrated under vaccum, diluted with water (5 ml), extracted with ethyl acetate (2x5ml) and dried over sodium sulphate. The combined fractions of ethyl acetate were concentrated under vaccum. The residue was chromatographed on silica gel using chloroform: hexane (20:80) as eluant to give **2p**; yield: 0.59 g. (81.7%), m.p. 110°C, C₁₇H₁₇BrN₂O₂; ¹H NMR δ ppm (CDCl₃): 1.94-2.00 (m, 2H), 2.77 (t, 2H), 2.87 (s, 3H), 3.20 (t, 2H), 6.53-6.57 (m, 1H), 6.78-6.87(m, 2H), 7.17-7.79 (m, 4H); IR νₘₐₓ (KBr) (cm⁻¹): 595, 674, 774, 883, 1004, 1194, 1411, 1479, 1270, 1595, 1742, 2382, 2927, 3431, 3689, 3774.

### Example 21: (4-Chloro-3-trifluoromethyl-phenyl)-carbamic acid 1-methyl-1,2,3,4-tetrahydro-quinolin-6-yl ester (2q)

(a) A solution of 1-methyl-1, 2, 3, 4-tetrahydro-quinolin-6-ol (0.326 g., 2 mmol) in dry tetrahydrofuran (5 ml) was added to a stirred suspension of sodium hydride (0.048 g., 2 mmol) in dry tetrahydrofuran (5 ml) at -10°C during 5 min. The reaction mixture was stirred for 30 min. Then 2-chloro-phenyl isocyanate (0.53, 2mmol) was added to the stirring reaction mixture. Stirring was continued for additional 2.5 hours during which the temperature was allowed to rise to room temperature (34°C). The reaction mixture was quenched with water (0.2 ml), concentrated under vaccum, diluted with water (5 ml), extracted with ethyl acetate (2x5ml) and dried over sodium sulphate. The combined fractions of ethyl acetate were concentrated under vaccum. residue was chromatographed on silica gel using chloroform: hexane (20:80) as eluant to give **2q**; yield: 0.52g. (67.6%), m.p. 152°C, C₁₈H₁₆ClF₃N₂O₂; ¹H NMR δ ppm (CDCl₃): 1.94-2.00 (m, 2H), 2.76 (t, 2H), 2.87 (s, 3H), 3.20 (t, 2H), 6.52-6.57 (m, 1H), 6.78-6.82(m, 2H), 7.46-7.81 (m, 3H); IR νₘₐₓ (KBr) (cm⁻¹): 537, 666, 756, 831, 894, 1029, 1147, 1264, 1423, 1489, 1542, 1596, 1698, 1741, 2373, 2928, 3118, 3232, 3402; 3687, 3760; MS: m/z: 385 (M⁺).
(b) A solution of 1 of (4-chloro-3-trifluoromethyl-phenyl)-carbamic acid -1,2,3,4- tetrahydro-quinolin-6-yl ester (0.74 g., 2 mmol) in dry dimethylformamide (5 ml) was added to a stirred suspension of sodium hydride (0.048 g., 2 mmol) in dry dimethylformamide (5 ml) at -10°C during 5 min. The reaction mixture was stirred for 30 min. Then methyl iodide (0.224 ml, 3.6 mmol) was added to the stirring reaction mixture. Stirring was continued for additional 1.25 hours during which the temperature was allowed to rise to room temperature (34°C). The reaction mixture was quenched with water (0.2 ml), concentrated under vaccum, diluted with water (5 ml), extracted with chloroform (2x5ml) and dried over sodium sulphate. The combined fractions of chloroform were concentrated under vaccum. The residue was chromatographed on silica gel using dichloromethane:hexane (20:80) as eluant to give 2q; yield: 0.42 g. (54.6%).

### Example 22. (4-bromo-phenyl)-carbamic acid 1-methyl-1, 2, 3, 4-tetrahydro-quinolin-6-yl ester (2r)

(a) A solution of 1-methyl-1, 2, 3, 4-tetrahydro-quinolin-6-ol (0.326 g., 2 mmol) in dry tetrahydrofuran (5 ml) was added to a stirred suspension of sodium hydride (0.048 g., 2 mmol) in dry tetrahydrofuran (5 ml) at -10°C during 5 min. The reaction mixture was stirred for 30 min. Then 2- bromo-phenyl isocyanate (0.734, 2mmol) was added to the stirring reaction mixture. Stirring was continued for additional 2.5 hours during which the temperature was allowed to rise to room temperature (34°C). The reaction mixture was quenched with water (0.2 ml), concentrated under vaccum, diluted with water (5 ml), extracted with ethyl acetate (2x5ml) and dried over sodium sulphate. The combined fractions of ethyl acetate were concentrated under vaccum. The residue was chromatographed on silica gel using chloroform: hexane (20:80) as eluant to give **2r**; yield: 0.40g. (52.3.%), m.p. 155°C, C₁₇H₁₇BrN₂O₂; ¹H NMR δ ppm (CDCl₃): 1.94-2:03 (m, 2H), 2.76 (t, 2H), 2.87 (s, 3H), 3.20 (t, 2H), 6.52-6.57 (m, 1H), 6.78-6.88(m, 2H), 7.25-7.45 (m, 4H); IR νₘₐₓ (KBr) (cm⁻¹): 674, 774, 1020, 1365, 1590, 2366, 2834, 2934, 3433, 3757, 3867, 3906; MS: m/z: 361 (M⁺).

## Claims

1. A substituted carbamic acid quinolinyl ester represented by formula 1 and 2 below: 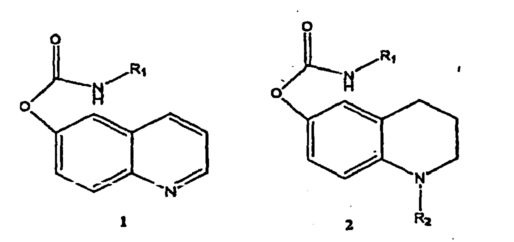 wherein R₁ = alkyl, aryl, 2-chlorophenyl, 3-bromo-phenyl, 4-bromophenyl, 4-chloro-3-trifluoromethyl-phenyl; R₂=H, alkyl, aralkyl.

2. A compound as claimed in claim 1 wherein the substituted carbamic acid quinolinyl ester is selected from the group consisting of:
**1a.** hexyl-carbamic acid quinolin-6-yl ester
**1b.** heptyl-carbamic acid quinolin-6-yl ester
**1c.** (2-chloro-phenyl)-carbamic acid quinolin-6-yl ester
**1d.** (3-bromo-phenyl)-carbamic acid quinolin-6-yl ester
**2a.** hexyl-carbamic acid 1, 2, 3,4-tetrahydro-quinolin-6-yl ester
**2b.** heptyl-carbamic acid 1, 2,3,4-tetrahydro-quinolin-6-yl ester
**2c.** (3-bromo-phenyl)-carbamic acid 1,2,3,4-tetrahydro-quinolin-6-yl ester
**2d.** (2-chloro-phenyl)-carbamic acid 1, 2, 3, 4-tetrahydro-quinolin-6-yl ester
**2e.** (4-chloro-3-trifluoromethyl-phenyl)-carbamic acid 1, 2, 3, 4-tetrahydro-quinolin-6-yl ester
**2f.** (4-bromo-phenyl)-carbamic acid 1, 2, 3, 4-tetrahydro-quinolin-6-yl ester
**2g.** heptyl-carbamic acid 1-benzyl-1, 2, 3, 4-tetrahydro-quinolin-6-yl ester
**2h.** hexyl-carbamic acid 1-benzyl-1, 2, 3, 4-tetrahydro-quinolin-6-yl ester
**2i.** (2-chloro-phenyl)-carbamic acid 1-benzyl-1, 2, 3, 4-tetrahydro-quinolin-6-yl ester
**2j.** (3-bromo-phenyl)-carbamic acid 1-benzyl-1, 2, 3, 4-tetrahydro-quinolin-6-yl ester
**2k** (4-chloro-3-trifluoromethyl-phenyl)-carbamic acid 1-benzyl-1, 2, 3, 4-tetrahydro-quinolin-6-yl ester
**2l.** (4-bromo-phenyl)-carbamic acid 1-benzyl-1, 2, 3, 4-tetrahydro-quinolin-6-yl ester
**2m.** hexyl-carbamic acid 1-methyl-1, 2, 3, 4-tetrahydro-quinolin-6-yl ester
**2n.** heptyl-carbamic acid 1-methyl-1, 2, 3, 4-tetrahydro-quinolin-6-yl ester
**2o.** (2-chloro-phenyl)-carbamic acid 1-methyl-1, 2, 3, 4-tetrahydro-quinolin-6-yl ester
**2p.** (3-bromo-phenyl)-carbamic acid 1-methyl-1, 2, 3, 4-tetrahydro-quinolin-6-yl ester
**2q**. (4-chloro-3-trifluoromethyl-phenyl)-carbamic acid 1-methyl-1, 2, 3, 4-tetrahydro-quinolin-6-yl ester
**2r**. (4-bromo-phenyl)-carbamic acid 1-methyl-1, 2, 3, 4-tetrahydro- quinolin-6-yl ester

3. A process for the synthesis of a quinoline derivative represented by formula **1** and **2** below: 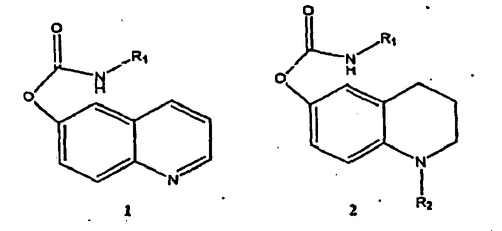 wherein R₁ = alkyl, aryl, 2-chlorophenyl, 3-bromo-phenyl, 4-bromophenyl, 4-chloro-3-trifluoromethyl-phenyl; R₂=H, alkyl, aralkyl, the process comprising reacting a substituted phenol with an isocyanate in the presence of a base and at least one organic solvent using a base and at least one organic solvent to obtain the corresponding carbamic acid ester (carbamates) of formulae 1 or 2.

4. A process as claimed in claim 3 wherein R₁ is selected from the group consisting of hexyl and heptyl.

5. A process as claimed in claim 3 wherein R₁ is selected from the group consisting of 2- chloro, 3- bromo, 4-bromo and 4-chloro-3-trifluoromethyl-phenyl.

6. A process as claimed in claim 3 wherein R₂ is selected from the group consisting of methyl and benzyl.

7. A process as claimed in claim 3 wherein the base used is selected from an organic or an inorganic base.

8. A process as claimed in claim 3 wherein solvent is selected from the group consisting of ether, tetrahydrofuran (THF), dimethylformamide (DMF), dioxane, dichloromethane and chloroform.

9. A process as claimed in claim 3 wherein the base is selected from the group consisting of sodium hydride, sodium hydroxide, triethylamine and pyridine.

10. A process as claimed in claim 3 wherein the reaction is carried out at a temperature in the range of -10°C to 80°C and for a period between half an hour to 100 hours.

11. A process as claimed in claim 3 wherein the reaction is carried out in the presence of a catalyst selected from the group consisting of sodium iodide and potassium iodide.

12. A process as claimed in claim 3 wherein the molar ratio of substituted phenol to isocyanate is in the range of 1:1 to 1:1.2.

13. A process as claimed in claim 3 wherein the organic solvent is present in an amount in the range of 1.0 ml to 10 ml per mmol of the reactants.

14. A process as claimed in claim wherein compound of formulae
**2g.** heptyl-carbamic acid 1-benzyl-1, 2, 3, 4-tetrahydro-quinolin-6-yl ester
**2h.** hexyl-carbamic acid 1-benzyl-1, 2, 3, 4-tetrahydro-quinolin-6-yl ester
**2i.** (2-chloro-phenyl)-carbamic acid 1-benzyl-1, 2, 3, 4-tetrahydro-quinolin-6-yl ester
**2j.** (3-bromo-phenyl)-carbamic acid 1-benzyl-1, 2, 3, 4-tetrahydro-quinolin-6-yl ester
**2k** (4-chloro-3-trifluoromethyl-phenyl)-carbamic acid 1-benzyl-1, 2, 3, 4-tetrahydro-quinolin-6-yl ester
**2l.** (4-bromo-phenyl)-carbamic acid 1-benzyl-1, 2, 3,4-tetrahydro-quinolin-6-yl ester
**2m.** hexyl-carbamic acid 1-methyl-1, 2, 3, 4-tetrahydro-quinolin-6-yl ester
**2n.** heptyl-carbamic acid 1-methyl-1, 2, 3, 4-tetrahydro-quinolin-6-yl ester
**2o.** (2-chloro-phenyl)-carbamic acid 1-methyl-1, 2, 3, 4-tetrahydro-quinolin-6-yl ester
**2p.** (3-bromo-phenyl)-carbamic acid 1-methyl-1, 2, 3, 4-tetrahydro-quinolin-6-yl ester
**2q.** (4-chloro-3-trifluoromethyl-phenyl)-carbamic acid 1-methyl-1, 2, 3, 4-tetrahydro-quinolin-6-yl ester
**2r.** (4·bromo-phenyl)-carbamic acid 1-methyl-1, 2, 3, 4-tetrahydro- quinolin-6-yl ester are obtained from **2a-f**
**2a,** hexyl-carbamic acid 1, 2, 3, 4-tetrahydro-quinolin-6-yl ester
**2b,** heptyl-carbamic acid 1, 2, 3, 4-tetrahydro-quinolin-6-yl ester
**2c.** (3-bromo-phenyl)-carbamic acid 1,2,3,4-tetrahydro-quinolin-6-yl ester
**2d.** (2-chloro-phenyl)-carbamic acid 1, 2, 3, 4-tetrahydro-quinolin-6-yl ester
**2e.** (4-chloro-3-trifluoromethyl-phenyl)-carbamic acid 1, 2, 3, 4-tetrahydro-quinolin-6-yl ester
**2f.** (4-bromo-phenyl)-carbamic acid 1, 2, 3, 4-tetrahydro-quinolin-6-yl ester
by first reacting compounds **2a-I** with an alkyl or aralkyl halide of formula RX wherein R is at least methyl or benzyl group and X is selected from chloro, bromo and iodio using a solvent selected from group consisting of dimethylformamide, tetrahydrofuran and dioxane, in the presence of an organic or inorganic base, selected from the group consisting of sodium hydride, sodium hydroxide, triethylamine and pyridine and at a temperature ranging between -10°C to 37°C for a period of 1 hour to 12 hours in the presence or absence of a catalyst sodium iodide or potassium iodide.

15. A process as claimed in claim 3 wherein compounds of formulae **2a-f** are synthesized from **2m-**r using Pd-C 5-10% catalyst in a solvent selected from group consisting of ethanol and methanol in an amount of 15-25 ml per mmol of compound, by applying hydrogen pressure in the range of 50-60 psi for a period between 4- 12 hours at room temperature.

16. A process as claimed in claim 3 wherein the phenol is reacted with an isocyanate to obtain corresponding carbamate which is then reduced with Ni- A1 alloy/KOH/ethanol to give corresponding 1, 2, 3, 4 - tetrahydro derivatives of formula **2a-l.**

17. A process as claimed in claim 3 wherein the phenol is reacted with an isocyanate to obtain corresponding N-benzyl derivatives **2m-r** which are then debenzylated using 5% or 10% Pd-C/H₂ in ethanol or methanol as solvent to obtain the corresponding debenzylated carbamates of formulae **2a-f,** which are then N-methylated using MeI to give compounds of formulae **2m-r.**

18. A process as claimed in claim 3 wherein compounds of formulae **2a-l** are methylated in the presence of a solvent selected from the group consisting of tetrahydrofuran, dioxane and dimethylformamide and at a temperature ranging from 10 to 37°C, for between 3 hours to 48 hours.

19. Use for the manufacture of a medicament for the treatment of hypofunctioning of cholinergic system in a subject suffering from hypofunctioning of cholinergic system, of a pharmaceutically effective amount of compound of formulae 1 or 2. 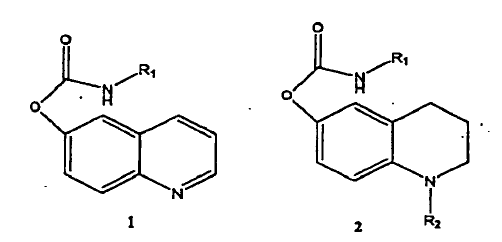 wherein R₁ = alkyl, aryl, 2-chlorophenyl, 3-bromo-phenyl, 4-bromophenyl, 4-chloro-3-trifluoromethyl-phenyl; R₂=H, alkyl, aralkyl.

20. Use as claimed in claim 19 wherein the hypofunctioning of cholinergic system occurs in the peripheral or central nervous system of the subject.

21. Use as claimed in claim 19 wherein the hypofunctioning of cholinergic system results in atony of smooth muscle of intestinal tract, atony of urinary bladder, glaucoma, myasthenia gravis and cognitive behaviour dysfunction of the subject.

22. Use as claimed in claim 19 wherein the subject is a mammal.

23. Use as claimed in claim 22 wherein the mammal is a human being.

## Patentansprüche

1. Substituierter Carbaminsäure-Quinolinylester, der nachfolgend durch Formel 1 und 2 dargestellt wird: 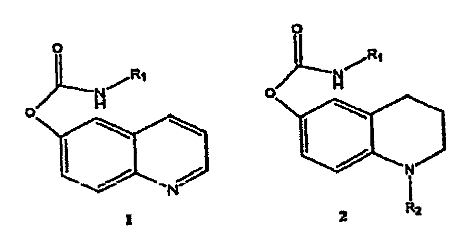 wobei R₁ = Alkyl, Aryl, 2-chlorphenyl, 3-bromphenyl, 4-bromphenyl, 4-chlor-3-trifluormethylphenyl; R₂ = H, Alkyl, Aralkyl.

2. Zusammensetzung nach Anspruch 1, wobei der substituierte Carbaminsäure-Quinolinylester ausgewählt ist aus der Gruppe bestehend aus:
1a. Hexyl-Carbaminsäure-Quinolin-6-yl-ester
1b. Heptyl-Carbaminsäure-Quinolin-6-yl-ester
1c. (2-chlorphenyl)-Carbaminsäure-Quinolin-6-yl-ester
1d. (3-bromphenyl)-Carbaminsäure-Quinolin-6-yl-ester
2a. Hexyl-Carbaminsäure-1,2,3,4-tetrahydro-Quinolin-6-yl-ester
2b. Heptyl-Carbaminsäure-1,2,3,4-tetrahydro-Quinolin-6-yl-ester
2c. (3-bromphenyl)-Carbaminsäure-1,2,3,4-tetrahydro-Quinolin-6-yl-ester
2d. (2-chlorphenyl)-Carbaminsäure-1,2,3,4-tetrahydro-Quinolin-6-yl-ester
2e. (4-chlor-3-trifluormethylphenyl)-Carbaminsäure-1,2,3,4-tetrahydro-Quinolin-6-yl-ester
2f. (4-bromphenyl)- Carbaminsäure-1,2,3,4-tetrahydro-Quinolin-6-yl-ester
2g. Heptyl-Carbaminsäure-1-benzyl-1,2,3,4-tetrahydro-Quinolin-6-yl-ester
2h. Hexyl-Carbaminsäure-1-benzyl-1,2,3,4-tetrahydro-Quinolin-6-yl-ester
2i. (2-chlorphenyl)-Carbaminsäure-1-benzyl-1,2,3,4-tetrahydro-Quinolin-6-yl-ester
2j. (3-bromphenyl)-Carbaminsäure-1-benzyl-1,2,3,4-tetrahydro-Quinolin-6-yl-ester
2k. (4-chlor-3-trifluormethylphenyl)-Carbaminsäure-1-benzyl-1,2,3,4-tetrahydro-Quinolin-6-yl-ester
2l. (4-bromphenyl)-Carbaminsäure-1-benzyl-1,2,3,4-tetrahydro-Quinolin-6-yl-ester
2m Hexyl-Carbaminsäure-1-methyl-1,2,3,4-tetrahydro-Quinolin-6-yl-ester
2n. Heptyl-Carbaminsäure-1-methyl-1,2,3,4-tetrahydro-Quinolin-6-yl-ester
2o. (2-chlorphenyl)-Carbaminsäure-1-methyl-1,2,3,4-tetrahydro-Quinolin-6-yl-ester
2p. (3-bromphenyl)-Carbaminsäure-1-methyl-1,2,3,4-tetrahydro-Quinolin-6-yl-ester
2q. (4-chlor-3-trifluormethylphenyl)-Carbaminsäure-1-methyl-1,2,3,4-tetrahydro-Quinolin-6-yl-ester
2r. (4-bromphenyl)-Carbaminsäure-1-methyl-1,2,3,4-tetrahydro-Quinolin-6-yl-ester

3. Verfahren zur Synthese eines Quinolinderivats, das nachfolgend durch Formel 1 und 2 dargestellt wird: 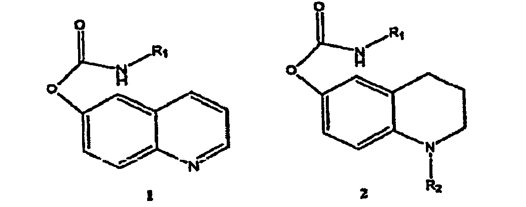 wobei R₁ = Alkyl, Aryl, 2-chlorphenyl, 3-bromphenyl, 4-bromphenyl, 4-chlor-3-trifluormethylphenyl; R₂ = H, Alkyl, Aralkyl, wobei das Verfahren die Reaktion eines substituierten Phenols mit einem Isocyanat in Anwesenheit einer Base und mindestens eines organischen Lösungsmittels umfasst, wobei eine Base und mindestens ein organisches Lösungsmittel verwendet wird, um den entsprechenden Carbaminsäureester (Carbamate) der Formeln 1 oder 2 zu erhalten.

4. Verfahren nach Anspruch 3, wobei R₁ ausgewählt ist aus der Gruppe bestehend aus Hexyl und Heptyl.

5. Verfahren nach Anspruch 3, wobei R₁ ausgewählt ist aus der Gruppe bestehend aus 2-chlor, 3-brom, 4-brom und 4-chlor-3-trifluormethylphenyl.

6. Verfahren nach Anspruch 3, wobei R₂ ausgewählt ist aus der Gruppe bestehend aus Methyl und Benzyl.

7. Verfahren nach Anspruch 3, wobei die verwendete Base ausgewählt ist aus einer organischen oder anorganischen Base.

8. Verfahren nach Anspruch 3, wobei das Lösungsmittel ausgewählt ist aus der Gruppe bestehend aus Ether, Tetrahydrofuran (THF), Dimethylformamid (DMF), Dioxan, Dichlormethan und Chloroform.

9. Verfahren nach Anspruch 3, wobei die Base ausgewählt ist aus der Gruppe bestehend aus Natriumhydrat, Natriumhydroxid, Triethylamin und Pyridin.

10. Verfahren nach Anspruch 3, wobei die Reaktion bei einer Temperatur im Bereich von -10°C bis 80°C und während einer Zeitdauer von einer halben Stunde bis 100 Stunden ausgeführt wird.

11. Verfahren nach Anspruch 3, wobei die Reaktion in Anwesenheit eines Katalysators ausgeführt wird, der ausgewählt ist aus der Gruppe bestehend aus Natriumiodid und Kaliumiodid.

12. Verfahren nach Anspruch 3, wobei das Molverhältnis des substituierten Phenols zum Isocyanat im Bereich von 1:1 bis 1:1,2 liegt.

13. Verfahren nach Anspruch 3, wobei das organische Lösungsmittel in einer Menge in einem Bereich von 1,0 ml bis 10 ml per mmol der Reaktionsmittel anwesend ist.

14. Verfahren nach Anspruch 3, wobei die Zusammensetzung der Formel
1a. Hexyl-Carbaminsäure-Quinolin-6-yl-ester
2g. Heptyl-Carbaminsäure-1-benzyl-1,2,3,4-tetrahydro-Quinolin-6-yl-ester
2h. Hexyl-Carbaminsäure-1-benzyl-1,2,3,4-tetrahydro-Quinolin-6-yl-ester
2i. (2-chlorphenyl)-Carbaminsäure-1-benzyl-1,2,3,4-tetrahydro-Quinolin-6-yl-ester
2j. (3-bromphenyl)-Carbaminsäure-1-benzyl-1,2,3,4-tetrahydro-Quinolin-6-yl-ester
2k. (4-chlor-3-trifluormethylphenyl)-Carbaminsäure-1-benzyl-1,2,3,4-tetrahydro-Quinolin-6-yl-ester
2l. (4-bromphenyl)-Carbaminsäure-1-benzyl-1,2,3,4-tetrahydro-Quinolin-6-yl-ester
2m. Hexyl-Carbaminsäure-1-methyl-1,2,3,4-tetrahydro-Quinolin-6-yl-ester
2n. Heptyl-Carbaminsäure-1-methyl-1,2,3,4-tetrahydro-Quinolin-6-yl-ester
2o. (2-chlorphenyl)-Carbaminsäure-1-methyl-1,2,3,4-tetrahydro-Quinolin-6-yl-ester
2p. (3-bromphenyl)-Carbaminsäure-1-methyl-1,2,3,4-tetrahydro-Quinolin-6-yl-ester
2q. (4-chlor-3-trifluormethylphenyl)-Carbaminsäure-1-methyl-1,2,3,4-tetrahydro-Quinolin-6-yl-ester
2r. (4-bromphenyl)-Carbaminsäure-1-methyl-1,2,3,4-tetrahydro-Quinolin-6-yl-ester erhalten werden von 2a-f
2a. Hexyl-Carbaminsäure-1,2,3,4-tetrahydro-Quinolin-6-yl-ester
2b. Heptyl-Carbaminsäure-1,2,3,4-tetrahydro-Quinolin-6-yl-ester
2c. (3-bromphenyl)-Carbaminsäure-1,2,3,4-tetrahydro-Quinolin-6-yl-ester
2d. (2-chlorphenyl)-Carbaminsäure-1,2,3,4-tetrahydro-Quinolin-6-yl-ester
2e. (4-chlor-3-trifluormethylphenyl)-Carbaminsäure-1,2,3,4-tetrahydro-Quinolin-6-yl-ester
2f. (4-bromphenyl)- Carbaminsäure-1,2,3,4-tetrahydro-Quinolin-6-yl-ester
wobei zuerst die Zusammensetzungen 2a-1 mit einem Alkyl- oder Aralkylhalid der Formel RX miteinander reagieren, wobei R mindestens eine Methyl- oder Benzylgruppe ist und X ausgewählt ist aus Chlor, Brom und Iod, wobei ein Lösungsmittel verwendet wird, das ausgewählt ist aus der Gruppe bestehend aus Dimethylformamid, Tetrahydrofuran und Dioxan, in Anwesenheit einer organischen oder anorganischen Base, die ausgewählt ist aus der Gruppe bestehend aus Natriumhydrid, Natriumhydroxid, Triethylamin und Pyridin und bei einer Temperatur im Bereich von -10°C bis 37°C während einer Zeitdauer von 1 Stunde bis 12 Stunden in Anwesenheit oder Abwesenheit eines Natriumiodid- oder Kaliumiodid-Katalysators.

15. Verfahren nach Anspruch 3, wobei die Zusammensetzungen der Formeln 2a-f von 2m-r synthetisiert werden, unter Anwendung eines Pd-C 5-10% Katalysators in einem Lösungsmittel, das ausgewählt ist aus der Gruppe bestehend aus Ethanol und Methanol in einer Menge von 15 bis 25 ml per mmol der Zusammensetzung, durch Anwenden eines Wasserstoffdrucks im Bereich von 50-60 psi für eine Zeitdauer von 4 bis 12 Stunden bei Zimmertemperatur.

16. Verfahren nach Anspruch 3, wobei das Phenol mit einem Isocyanat reagiert, um ein entsprechendes Carbamat zu erhalten, das sodann mit einem Ni-Al-Gemisch/KOH/Ethanol reduziert wird, um entsprechende 1,2,3,4-tetrahydro-Derivate der Formel 2a-1 zu ergeben.

17. Verfahren nach Anspruch 3, wobei das Phenol mit einem Isocyanat reagiert, um entsprechende N-Benzyl-Derivate 2m-r zu erhalten, die sodann unter Anwendung von 5% oder 10% Pd-C/H₂ in Ethanol oder Methanol als Lösungsmittel debenzylisiert werden, um die entsprechenden debenzylierten Carbamate der Formeln 2a-f zu erhalten, die sodann unter Anwendung von MeI N-methyliert werden, um Zusammensetzungen der Formeln 2m-r zu ergeben.

18. Verfahren nach Anspruch 3, wobei die Zusammensetzungen der Formeln 2a-1 in Anwesenheit eines Lösungsmittels, ausgewählt aus der Gruppe bestehend aus Tetrahydrofuran, Dioxan und Dimethylformamid, methylisiert werden, und dies, bei einer Temperatur im Bereich von 10 bis 37°C während 3 bis 48 Stunden.

19. Anwendung, zur Herstellung eines Arzneimittels zur Behandlung einer Unterfunktion des cholinergischen Systems bei einem Patienten, der an einer Unterfunktion des cholinergischen Systems leidet, einer pharmazeutisch akzeptablen Menge der Zusammensetzung der Formeln 1 oder 2: 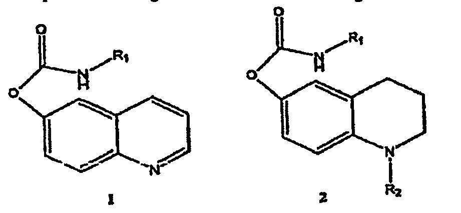 wobei R₁ = Alkyl, Aryl, 2-chlorphenyl, 3-bromphenyl, 4-bromphenyl, 4-chlor-3-trifluormethylphenyl; R₂ = H, Alkyl, Aralkyl.

20. Anwendung nach Anspruch 19, wobei die Unterfunktion des cholinergischen Systems im peripheren Nervensystem oder im Zentralnervensystem des Patienten stattfmdet.

21. Anwendung nach Anspruch 19, wobei die Unterfunktion des cholinergischen Systems zu Folgendem führt: Atonie der glatten Muskulatur des Darmtrakts, Atonie der Harnblase, Glaukom, Myasthenia gravis und kognitive Verhaltensdysfunktion des Patienten.

22. Anwendung nach Anspruch 19, wobei der Patient ein Säuger ist.

23. Anwendung nach Anspruch 22, wobei der Säuger ein Mensch ist.

## Revendications

1. Ester quinolinylique d'acide carbamique substitué représenté par les formules 1 et 2 ci-dessous: 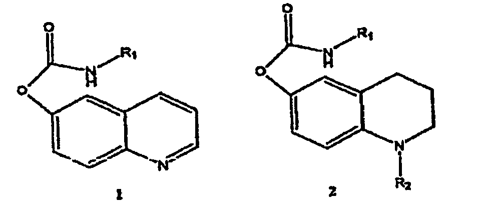 dans lequel R₁=alkyl, aryl, 2-chlorophényl, 3-bromo-phényl, 4-bromophényl, 4-chloro-3-trifluorométhyl-phényl; R₂=H, alkyl, aralkyl.

2. Composé selon la revendication 1, dans lequel l'ester quinolinylique d'acide carbamique substitué est sélectionné dans le groupe consistant en:
1a. ester quinolin-6-ylique d'acide hexyl-carbamique
1b ester quinolin-6-ylique d'acide heptyl-carbamique
1c. ester quinolin-6-ylique d'acide (2-chloro-phényl)-carbamique
1d. ester quinolin-6-ylique d'acide (3-bromo-phényl)-carbamique
2a. ester 1, 2, 3, 4-tétrahydro-quinolin-6-ylique d'acide hexyl-carbamique
2b. ester 1, 2, 3, 4-tétrahydro-quinolin-6-ylique d'acide heptyl-carbamique
2c. ester 1,2,3,4-tétrahydro-quinolin-6-ylique d'acide (3-bromo-phényl)-carbamique
2d. ester 1, 2, 3, 4-tétrahydro-quinolin-6-ylique d'acide (2-chloro-phényl)-carbamique
2e. ester 1, 2, 3, 4-tétrahydro-quinolin-6-ylique d'acide (4-chloro-3-trifluorométhyl-phényl)-carbamique
2f. ester 1, 2, 3, 4-tétrahydro-quinolin-6-ylique d'acide (4-bromo-phényl)-carbamique
2g. ester 1 -benzyl- 1, 2, 3, 4-tétrahydro-quinolin-6-ylique d'acide heptyl-carbamique
2h. ester 1-benzyl- 1, 2, 3, 4-tétrahydro-quinolin-6-ylique d'acide hexyl-carbamique
2i. ester 1-benzyl-1, 2, 3, 4-tétrahydro-quinolin-6-ylique d'acide (2-chloro-phényl)-carbamique
2j. ester 1-benzyl-1, 2, 3, 4-tétrahydro-quinolin-6-ylique d'acide (3-bromo-phényl)-carbamique
2k. ester 1-benzyl-1, 2, 3, 4-tétrahydro- quinolin-6-ylique d'acide (4-chloro-3-trifluorométhyl-phényl)-carbamique
2l. ester 1-benzyl-1, 2, 3, 4-tétrahydro-quinolin-6-ylique d'acide (4-bromo-phényl)-carbamique
2m. ester 1-méthyl-1, 2, 3, 4-tétrahydro-quinolin-6-ylique d'acide hexyl-carbamique
2n. ester 1-méthyl-1, 2, 3, 4-tétrahydro-quinolin-6-ylique d'acide heptyl-carbamique
2o. ester 1-méthyl-1, 2, 3, 4-tétrahydro-quinolin-6-ylique d'acide (2-chloro-phényl)-carbamique
2p. ester 1-méthyl-1, 2, 3, 4-tétrahydro-quinolin-6-ylique d'acide (3-bromo-phényl)-carbamique
2q. ester 1-méthyl-1, 2, 3, 4-tétrahydro-quinolin-6-ylique d'acide (4-chloro-3-trifluorométhyl-phényl)-carbamique
2r. ester 1-méthyl-1, 2, 3, 4-tétrahydro- quinolin-6-ylique d'acide (4-bromo-phényl)-carbamique.

3. Procédé pour la synthèse d'un dérivé de quinoline représenté par les formules 1 et 2 ci-dessous : 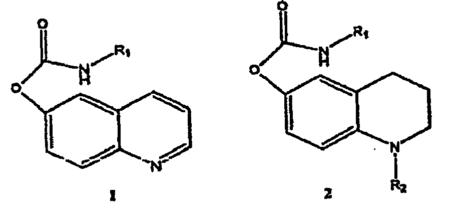 où R₁=alkyl, aryl, 2-chlorophényl, 3-bromo-phényl, 4-bromophényl, 4-chloro-3-trifluorométhyl-phényl ; R₂=H, alkyl, aralkyl, le procédé comprenant la mise en réaction d'un phénol substitué avec un isocyanate en présence d'une base et d'au moins un solvant organique en utilisant une base et au moins un solvant organique pour obtenir l'ester d'acide carbamique correspondant (carbamates) des formules 1 ou 2.

4. Procédé selon la revendication 3, dans lequel R₁ est sélectionné dans le groupe consistant en hexyl et heptyl.

5. Procédé selon la revendication 3, dans lequel R₁ est sélectionné dans le groupe consistant en 2-chloro, 3-bromo, 4-bromo et 4-chloro-3-trifluorométhyl-phényl.

6. Procédé selon la revendication 3, dans lequel R₂ est sélectionné dans le groupe consistant en méthyl et benzyl.

7. Procédé selon la revendication 3, dans lequel la base utilisée est sélectionnée à partir d'une base organique ou inorganique.

8. Procédé selon la revendication 3, dans lequel le solvant est sélectionné dans le groupe consistant en éther, tétrahydrofurane (THF), diméthylformamide (DMF), dioxane, dichlorométhane et chloroforme.

9. Procédé selon la revendication 3, dans lequel la base est sélectionnée dans le groupe consistant en hydrure de sodium, hydroxyde de sodium, triéthylamine et pyridine.

10. Procédé selon la revendication 3, dans lequel la réaction est effectuée à une température allant de -10°C à 80°C et pour une période allant d'une demi-heure à 100 heures.

11. Procédé selon la revendication 3, dans lequel la réaction est effectuée en présence d'un catalyseur sélectionné dans le groupe consistant en iodure de sodium et iodure de potassium.

12. Procédé selon la revendication 3, dans lequel le rapport molaire du phénol substitué à l'isocyanate est dans la plage de 1:1 à 1:1,2.

13. Procédé selon la revendication 3, dans lequel le solvant organique est présent dans une quantité allant de 1,0 ml à 10 ml par mmol des réactifs.

14. Procédé selon la revendication 3, dans lequel les composés des formules
2g. ester 1 -benzyl- 1, 2, 3, 4-tétrahydro-quinolin-6-ylique d'acide heptyl-carbamique
2h. ester 1-benzyl-1, 2, 3, 4-tétrahydro-quinolin-6-ylique d'acide hexyl-carbamique
2i. ester 1-benzyl-1, 2, 3, 4-tétrahydro-quinolin-6-ylique d'acide (2-chloro-phényl)-carbamique
2j. ester 1-benzyl-1, 2, 3, 4-tétrahydro-quinolin-6-ylique d'acide (3-bromo-phényl)-carbamique
2k. ester 1-benzyl-1, 2, 3, 4-tétrahydro- quinolin-6-ylique d'acide (4-chloro-3-trifluorométhyl-phényl)-carbamique
2l. ester 1-benzyl-1, 2, 3, 4-tétrahydro-quinolin-6-ylique d'acide (4-bromo-phényl)-carbamique
2m. ester 1 -méthyl- 1, 2, 3, 4-tétrahydro-quinolin-6-ylique d'acide hexyl-carbamique
2n. ester 1-méthyl-1, 2, 3, 4-tétrahydro-quinolin-6-ylique d'acide heptyl-carbamique
2o. ester 1-méthyl-1, 2, 3, 4-tétrahydro-quinolin-6-ylique d'acide (2-chloro-phényl)-carbamique
2p. ester 1-méthyl-1, 2, 3, 4-tétrahydro-quinolin-6-ylique d'acide (3-bromo-phényl)-carbamique
2q. ester 1-méthyl-1, 2, 3, 4-tétrahydro-quinolin-6-ylique d'acide (4-chloro-3-trifluorométhyl-phényl)-carbamique
2r. ester 1-méthyl-1, 2, 3, 4-tétrahydro-quinolin-6-ylique d'acide (4-bromo-phényl)-carbamique sont obtenus à partir de 2a-f
2a. ester 1, 2, 3, 4-tétrahydro-quinolin-6-ylique d'acide hexyl-carbamique
2b. ester 1, 2, 3, 4-tétrahydro-quinolin-6-ylique d'acide heptyl-carbamique
2c. ester 1, 2, 3, 4-tétrahydro-quinolin-6-ylique d'acide (3-bromo-phényl)-carbamique
2d. ester 1, 2, 3, 4-tétrahydro-quinolin-6-ylique d'acide (2-chloro-phényl)-carbamique
2e. ester 1, 2, 3, 4-tétrahydro-quinolin-6-ylique d'acide (4-chloro-3-trifluorométhyl-phényl)-carbamique
2f. ester 1, 2, 3, 4-tétrahydro-quinolin-6-ylique d'acide (4-bromo-phényl)-carbamique
en faisant tout d'abord réagir les composés 2a-1 avec un halogénure d'alkyle ou d'aralkyle de la formule RX où R est au moins un groupe méthyl ou benzyl et X est sélectionné parmi chloro, bromo et iodio en utilisant un solvant sélectionné dans le groupe consistant en diméthylformamide, tétrahydrofurane et dioxane, en présence d'une base organique ou inorganique sélectionnée dans le groupe consistant en hydrure de sodium, hydroxide de sodium, triéthylamine et pyridine et à une température allant de -10°C à 37°C pour une période d'une heure à 12 heures en présence ou en absence d'un catalyseur iodure de sodium ou iodure de potassium.

15. Procédé selon la revendication 3, dans lequel les composés des formules 2a-f sont synthétisés à partir de 2m-r en utilisant un catalyseur Pd-C 5-10% dans un solvant sélectionné dans le groupe consistant en éthanol et méthanol dans une quantité de 15-25 ml par mmol de composé, en appliquant une pression d'hydrogène de l'ordre de 50-60 psi pour une période de 4 à 12 heures à température ambiante.

16. Procédé selon la revendication 3, dans lequel le phénol est mis en réaction avec un isocyanate pour obtenir le carbamate correspondant qui est alors réduit avec alliage Ni-Al/KOH/éthanol pour donner les dérivés 1, 2, 3, 4-tétrahydro des formules 2a-1.

17. Procédé selon la revendication 3, dans lequel le phénol est mis en réaction avec un isocyanate pour obtenir les dérivés 2m-r N-benzyl correspondants qui sont alors débenzylés en utilisant 5% ou 10% Pd-C/H₂ dans de l'éthanol ou du méthanol comme solvant pour obtenir les carbamates débenzylés des formules 2a-f, qui sont ensuite N-méthylés en utilisant MeI pour donner les composés des formules 2m-r.

18. Procédé selon la revendication 3, dans lequel les composés des formules 2a-l sont méthylés en présence d'un solvant sélectionné dans le groupe consistant en tétrahydrofurane, dioxane et diméthylformamide et à une température allant de 10 à 37°C pendant 3 à 48 heures.

19. Utilisation, pour la fabrication d'un médicament pour le traitement du fonctionnement déficient du système cholinergique chez un sujet souffrant d'un fonctionnement déficient du système cholinergique, d'une quantité pharmaceutiquement efficace du composé des formules 1 ou 2: 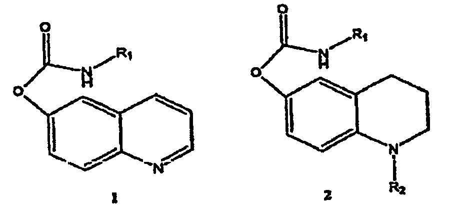 où R₁=alkyl, aryl, 2-chlorophényl, 3-bromo-phényl, 4-bromophényl, 4-chloro-3-trifluorométhyl-phényl; R₂=h, alkyl, aralkyl.

20. Utilisation selon la revendication 19, dans laquelle le fonctionnement déficient du système cholénergique a lieu dans le système nerveux central ou périphérique du sujet.

21. Utilisation selon la revendication 19, dans laquelle le fonctionnement déficient du système cholinergique résulte en une atonie du muscle lisse du tractus intestinal, atonie de la vessie, glaucome, myasthénie grave et dysfonction comportementale cognitive du sujet.

22. Utilisation selon la revendication 19, dans laquelle le sujet est un mammifère.

23. Utilisation selon la revendication 22, dans laquelle le mammifère est un être humain.
